# EUROPEAN PATENT SPECIFICATION

(11) **EP 3 300 654 B1**
(45) Date of publication and mention of the grant of the patent: **05.01.2022**
(21) Application number: 17194187.5
(22) Date of filing: 29.09.2017
(51) Int. Cl.: A61B 3/12

(54) **METHOD FOR FUNDUS IMAGE OBSERVATION**
FUNDUSBILDBEOBACHTUNGSMETHODE
MÉTHODE D'OBSERVATION D'UNE IMAGE DE FOND D'OEIL

(30) Priority: 30.09.2016 JP 2016194511; 30.09.2016 JP 2016194512; 30.09.2016 JP 2016194513
(43) Date of publication of application: 04.04.2018
(73) Proprietor: Nidek Co., Ltd., Gamagori-shi, Aichi, 443-0038 (JP)
(72) Inventor: Fujiu, Kenshiro, Gamagori-shi, Aichi 443-0038 (JP)
(74) Representative: Zimmermann, Tankred Klaus

(56) References cited:
- JP-A- 2005 006 894
- US-A1- 2008 030 684
- US-A1- 2015 002 812

## Description

### BACKGROUND

### 1. Technical Field

The present disclosure relates to a method for fundus image observation using a processor of a computer and a fundus image observation program that at least displays a fundus front image using fluorescence, the fundus front image having been obtained by fluorescent angiography.

### 2. Description of Related Art

A fluorescent angiography described below is typically performed mainly in the ophthalmic field. In the angiography, firstly, a fluorescent contrast agent such as fluorescein or indocyanine green is injected into a blood vessel of an examinee. The fluorescent contrast agent that has circulated into the examinee's eye is irradiated with excitation light to chronologically photograph the fundus that emits fluorescence (refer to, for example, JP-A-2016-59400). For example, a lesion is difficult to be found from the state of the flow of blood and in a normal fundus examination is observed and diagnosed by an examiner from a contrast medium appearance of the fluorescent contrast agent in the fundus, the contrast medium appearance being rendered in chronologically photographed fundus front images of a plurality of frames.

Images required for the observation, diagnosis, and the like of the examinee's eye among the fundus front images of the plurality of frames obtained as a result of the fluorescent imaging procedure may be only a part of the plurality of frames. In this case, it is conceivable that only a part, which has been selected by the examiner, of the fundus front images of the plurality of frames obtained through the fluorescent imaging procedure is displayed on a display unit. Hence, a technique for suitably displaying an image selected by the examiner from the fundus front images obtained by fluorescent angiography has been studied.

Moreover, fluorescent angiography is performed over a long time, approximately several tens of minutes. In the early stage of the angiography, the contrast medium appearance changes quickly. On the other hand, the contrast medium appearance changes less and more gently in the middle and later stages of the angiography than in the early stage of the angiography. Hence, it is conceivable that photographing is performed, dividing the session into several sessions according to the angiography timings after the contrast agent is injected into a vein. In this case, image data including one or more frame images is generated in each photography session. However, it is required to select image data and then check images included in the image data to check a photographing result after the fluorescent imaging procedure, which is a burden on the examiner.

Furthermore, a thorough study has not been typically conducted on the layout of fluorescent angiographic images of a plurality of frames used for the observation, diagnosis, and the like of an examinee's eye.

The present disclosure has been made considering the above-described circumstances. In other words, a technical issue in the present disclosure is to provide a fundus image observation program that causes an image that is selected by an examiner from fundus front images obtained by fluorescent angiography to be suitably displayed.

Moreover, the present disclosure has a technical issue to provide a fundus image observation program that is easy to reduce a burden on an examiner when an image obtained through a fluorescent imaging procedure is checked.

Furthermore, the present disclosure has a technical issue to provide a fundus image observation program that causes fundus front images obtained by fluorescent angiography to be displayed and printed in suitable layout.

US 2008/0030684 A1 discloses an ophthalmologic imaging system in which images are acquired from an eye of an examinee. US 2008/0030684 A1 discloses selecting one image which is to be enlarged and displayed from thumbnails of a plurality of fluorescent angiographic images.

### SUMMARY

The invention is defined in the appended claims.

A fundus image observation program according to aspects of the present disclosure is as follows.
(1) A fundus image observation program causing a processor of a computer to execute:
   a first acquisition step of acquiring image group data including fluorescent angiographic images of a plurality of frames acquired through a fluorescent imaging procedure for an examinee's eye; a second acquisition step of selecting and setting at least one or more frames illustrating a desired contrast medium appearance as key frames from the fluorescent angiographic images included in the image group data and obtaining key frame specification information for specifying the key frames in the image group data;
   and a list display step of displaying the key frames extracted from the image group data in list form on a display unit on the basis of the key frame specification information.
(2) The fundus image observation program according to (1), wherein in the first acquisition step, the image group data is acquired which includes at least a photographed image group being the fluorescent angiographic images of a plurality of frames photographed continuously at regular intervals, the image group data further includes the fluorescent angiographic image photographed at an output timing of a third trigger signal emitted during photographing of the photographed image data, in the second acquisition step, the fluorescent angiographic image photographed at the output timing of the third trigger signal, the fluorescent angiographic image being included in the photographed image group, is selected and set as the key frame or a candidate therefor.
(3) The fundus image observation program according to (2), wherein the image group data includes the photographed image group and a still image of the fluorescent angiographic image photographed singly at each output timing of a photographing trigger signal, and in the second acquisition step, the key frame specification information of the photographed image group is acquired and also all the still images included in the image group data are selected and set as the key frames or candidates therefor.
(4) The fundus image observation program according to any one of (1) to (3), wherein in the second acquisition step, a key frame selection screen for selecting the key frames from the fluorescent angiographic images of the plurality of frames included in the image group data on the basis of an examiner's selection operation is displayed on the display unit, at least one or more frames of the fluorescent angiographic images included in the image group data are displayed on the key frame selection screen, and at least one or more fluorescent angiographic images in accordance with the selection operation are selected and set as the key frames from the fluorescent angiographic images displayed on the key frame selection screen.
(5) The fundus image observation program according to (4), wherein in the second acquisition step, a selection operation of selecting an angiography timing via an input interface of the computer is accepted, and the fluorescent angiographic image photographed at the angiography timing selected by the selection operation is extracted on the basis of the image group data, and displayed on the key frame selection screen.
(6) The fundus image observation program according to (4) or (5), wherein in the first acquisition step, the image group data is acquired which includes at least the photographed image group being the fluorescent angiographic images of the plurality of frames photographed continuously at regular intervals, and in the second acquisition step, frame images are extracted from the photographed image group, a widget for selecting a photographing time interval, a frame interval, or an extraction number of the frame images is displayed on the key frame selection screen, and the fluorescent angiographic images extracted at the photographing time intervals, at the frame intervals, or in the extraction number on the basis of an input operation via the widget are displayed on the key frame selection screen.
(7) The fundus image observation program according to any one of (4) to (6), wherein in the first acquisition step, the image group data is acquired which includes at least the photographed image group being the fluorescent angiographic images of the plurality of frames photographed continuously at regular intervals, and in the second acquisition step, the photographed image group is switched and displayed on the key frame selection screen, and a fourth trigger signal emitted during the switching and display is accepted to select and set, as the key frame, the fluorescent angiographic image displayed at an output timing of the fourth trigger signal.
(8) The fundus image observation program according to any one of (4) to (7) causing the computer to execute a displacement correction step of correcting displacements of photographing positions of the fluorescent angiographic images included in the image group data, wherein in the second acquisition step, the fluorescent angiographic image after the displacement correction is displayed on the key frame selection screen.
(9) The fundus image observation program according to any one of (4) to (8), wherein in the second acquisition step, thumbnails of the fluorescent angiographic images are arranged and displayed in photographing order in a thumbnail display area of the key frame selection screen, and upon any of the thumbnails displayed in the thumbnail display area being selected as a detailed display target, the fluorescent angiographic image corresponding to the selected thumbnail is displayed in a larger size or with a higher number of pixels than the thumbnail.
(10) The fundus image observation program according to any one of (1) to (9), wherein in the list display step, upon input of a predetermined operation on a first key frame being any of the key frames displayed on the display unit having been accepted via the input interface of the computer, frames included in the image group data other than the key frames, the frames being within a predetermined period from an angiography timing of the first key frame, are displayed on the display unit on the basis of the image group data.
(11) The fundus image observation program according to (10) causing the computer to further execute a reselection step, wherein in the reselection step, upon in the list display step, the frames within the predetermined period from the angiography timing of the first key frame being displayed on the basis of the input of the predetermined operation, any of the frames within the predetermined period is selected and set as a new key frame on the basis of a second selection operation via the input interface of the computer.
(12) The fundus image observation program according to any one of (1) to (11), causing the computer to further execute a determination step of determining whether or not each of the fluorescent angiographic images of the plurality of frames included in the image group data is a failed photographed image photographed at a timing of occurrence of a blink or fixation disparity, wherein in the second acquisition step, the failed photographed image among the fluorescent angiographic images of the plurality of frames included in the image group data is excluded from the key frames and candidates therefor.
(13) The fundus image observation program according to any one of (1) to (12), wherein the image group data acquired in the first acquisition step includes elapsed time information indicating time elapsed in the fluorescent imaging procedure, the time elapsing at a photographing timing of each frame included in the image group data, and in the list display step, the elapsed time is displayed, associated with each key frame displayed in list form on the display unit on the basis of the elapsed time information.
(14) A fundus image observation program that at least displays fluorescent angiographic images being fundus front images photographed in fluorescent angiography with a fundus photographing apparatus, the fundus image observation program causing a processor of a computer to execute: an acquisition step of acquiring at least one first fluorescent angiographic image photographed on the basis of a first photographing trigger signal, and at least one second fluorescent angiographic image photographed on the basis of a second photographing trigger signal whose output timing is different from the first photographing trigger signal as one set of image group data through a fluorescent imaging procedure for an examinee's eye; and a display step of causing a display unit to selectively display the fluorescent angiographic image photographed at any angiography timing from the first and second fluorescent angiographic images included in the image group data on the basis of the image group data.
(15) The fundus image observation program of (14), wherein the first fluorescent angiographic images are images of a plurality of frames photographed continuously at regular intervals after an output timing of the first photographing trigger signal, the second fluorescent angiographic images are still images photographed singly on the basis of the second photographing trigger signal, and in the display step, angiography timings of the first fluorescent angiographic images and angiography timings of the second fluorescent angiographic images are assumed to be one time series, and any angiography timing is selected in the time series, and displayed on the display unit.
(16) The fundus image observation program of (15), wherein in the display step, an identification graphic for distinguishing the first and second fluorescent angiographic images is displayed, associated with at least one of the first and second fluorescent angiographic images.
(17) The fundus image observation program described in any one of (14) to (16), wherein in the display step, a widget for accepting an operation of selecting the angiography timing is displayed on the display unit, and the fluorescent angiographic image at the angiography timing selected from the image group data is displayed on the display unit on the basis of an operation on the widget.
(18) The fundus image observation program of (17), wherein in the display step, the widget is displayed as a slider corresponding to the time series including the angiography timings of the first and second fluorescent angiographic images.
(19) The fundus image observation program described in any one of (14) to (18), wherein in the display step, images of a plurality of frames whose angiography timings are close to each other are extracted from the first and second fluorescent angiographic images included in the one set of image group data in accordance with the selection operation, and the extracted images are arranged and displayed in photographing order.
(20) The fundus image observation program of any one of (14) to (19), wherein in the display step, thumbnails of the first and second fluorescent angiographic images included in the image group data are arranged and displayed in photographing order in a thumbnail display area, and when any of the thumbnails displayed in the thumbnail display area is selected as a detailed display target, the fluorescent angiographic image corresponding to the selected thumbnail is displayed in a larger size or with a higher number of pixels than the thumbnail.
(21) The fundus image observation program of (20), wherein the first fluorescent angiographic images are images of a plurality of frames photographed continuously at regular intervals after the output timing of the first photographing trigger signal, and when the first fluorescent angiographic image is selected as the detailed display target in the display step, the images of the plurality of frames can be switched and displayed in photographing order.
(22) The fundus image observation program of (20) or (21), wherein in the display step, an identification graphic for distinguishing the thumbnail selected as the detailed display target and other thumbnails is displayed, associated with the selected thumbnail.
(23) The fundus image observation program of any one of (14) to (22), wherein the first and second fluorescent angiographic images are images of a plurality of frames photographed continuously at regular intervals after the signal output timings of the first and second photographing trigger signals, and in the display step, the first and second fluorescent angiographic images are extracted automatically at least one frame at a time according to the first and second photographing trigger signals, and the extracted frames are displayed.
(24) The fundus image observation program of any one of (14) to (23), wherein the first fluorescent angiographic images are images of a plurality of frames photographed continuously at regular intervals after the output timing of the first photographing trigger signal, and in the display step, images are extracted at predetermined photographing time intervals, at predetermined frame intervals, or a predetermined number of images at a time, from the plurality of first fluorescent angiographic images photographed on the basis of the first photographing trigger signal, and the extracted frames are displayed.
(25) The fundus image observation program of (24), wherein in the display step, a second widget for selecting the photographing time interval, the frame interval, or the extraction number of frame images extracted from the plurality of first fluorescent angiographic images is displayed on the display unit, and the first fluorescent angiographic images extracted at the photographing time intervals, at the frame intervals, or in the extraction number on the basis of an input operation via the second widget are displayed.
(26) The fundus image observation program of any one of (14) to (25), wherein the first fluorescent angiographic images are images of a plurality of frames photographed continuously at regular intervals after the output timing of the first photographing trigger signal, in the acquisition step, specification information is further acquired which specifies the first fluorescent angiographic image photographed at an output timing of a third trigger signal emitted while the first fluorescent angiographic images of the plurality of frames are photographed on the basis of the first photographing trigger signal, and in the display step, an image specified by the specification information is selectively displayed from the first fluorescent angiographic images included in the image group data.
(27) The fundus image observation program of any one of (14) to (26), wherein the first fluorescent angiographic images are images of a plurality of frames photographed continuously at regular intervals after the output timing of the first photographing trigger signal, the fundus image observation program causes the computer to further execute an acquisition step of switching and displaying the first fluorescent angiographic images included in the image group data acquired in advance, on the display unit, on the basis of the image group data, and also accepting a fourth trigger signal emitted during the switching and display to acquire second specification information that specifies the first fluorescent angiographic image displayed at an output timing of the fourth trigger signal, and in the display step, and an image specified by the second specification information is selectively displayed from the first fluorescent angiographic images included in the image group data.
(28) The fundus image observation program of any one of (14) to (27) causing the computer to further execute a determination step of determining whether or not each fluorescent angiographic image included in the image group data is a failed photographed image photographed at a timing when a blink or fixation disparity occurs, wherein in the display step, the fluorescent angiographic images are displayed on the display unit after the failed photographed image is excluded in advance.
(29) The fundus image observation program of any one of (14) to (28) causing the computer to execute a displacement correction step of correcting displacements of photographing positions of the fluorescent angiographic images included in the image group data, wherein in the display step, the fluorescent angiographic image after the displacement correction is displayed.
(30) The fundus image observation program of (29) causing the computer to further execute a highlight step of setting a region of interest in any one of the first and second fluorescent angiographic images included in the image group data on the basis of an input operation on an input interface of the computer, and applying a highlight that emphasizes the set region of interest when displaying another fluorescent angiographic image after the displacement correction included in the image group data.
(31) The fundus image observation program of any one of (14) to (30), wherein in the acquisition step, time information indicating an angiography timing of each fluorescent angiographic image is acquired, and in the display step, the time information of each fluorescent angiographic image displayed on the display unit, together with the image, is displayed.
(32) A fundus image observation program causing a processor of a computer to execute:
   an acquisition step of acquiring a plurality of first fluorescent angiographic images photographed with an ophthalmologic photographing apparatus, and a plurality of second fluorescent angiographic images photographed with the ophthalmologic photographing apparatus, the plurality of second fluorescent angiographic images being different from the first fluorescent angiographic images in any of type of angiography,
   angiography timing, and photographing date; a summary edition step of displaying, on a display unit, a summary edition area for an edition as a summary for observation or
   diagnosis of an examinee's eye, and also selecting at least one first fluorescent angiographic image illustrating a desired contrast medium appearance, and at least one second fluorescent angiographic image illustrating the desired contrast medium appearance, and arranging and placing the selected images in sub areas different from each other among a plurality of sub areas in the summary edition area; and a summary output step of displaying the summary edited in the summary edition step on the display unit, or printing the summary.
(33) The fundus image observation program of (32), wherein in the summary edition step, a selection area where the first and second fluorescent angiographic images acquired in the acquisition step are listed is displayed simultaneously with the summary edition area, and also at least one of each of the first and second fluorescent angiographic images is selected from the images listed in the selection area to accept a placement operation of placing the images in their corresponding sub areas.
(34) The fundus image observation program of (32) or (33), wherein the first and second fluorescent angiographic images are images that are different in type of angiography from each other, and in the summary edition step, in terms of the first or second fluorescent angiographic image placed in the sub area, the image on display is switched to an image whose angiography timing is close in fluorescent angiography and whose type of angiography is the same in accordance with a predetermined operation on an input interface.
(35) The fundus image observation program of (34), wherein in the summary edition step, a widget is displayed, associated with the sub area where the first or second fluorescent angiographic image is placed, and an one-action operation on the widget is accepted as the predetermined operation to switch and display the fluorescent angiographic images in the sub area corresponding to the operated widget on the basis of the operation.
(36) The fundus image observation program of (32) or (33), wherein the first and second fluorescent angiographic images are images photographed in the early stage of an angiography and the middle and later stages of the angiography through a fluorescent imaging procedure, and in the acquisition step, a reflection image based on reflected light from the fundus of the examinee's eye, or an autofluorescence image by autofluorescence from the fundus is acquired, and the reflection image or the autofluorescence image is displayed, in parallel with the two sub areas, in a third sub area being the sub area different from the two sub areas where the first and second fluorescent angiographic images are placed respectively.
(37) The fundus image observation program of (32) or (33), wherein the first and second fluorescent angiographic images are images that are photographed on photographing dates different from each other, and that are the same in type of angiography.
(38) The fundus image observation program of (37), wherein in the acquisition step, information on the angiography timing of each of the first and second fluorescent angiographic images, together with the image, is acquired, and in the summary edition step, a first edition area where sub areas where the first fluorescent angiographic image can be placed are arranged in a first direction, and a second edition area where sub areas where the second fluorescent angiographic image can be placed are arranged in the first direction are arranged in a second direction crossing the first direction in the summary edition area, and when the first fluorescent angiographic image is placed in a first sub area being one of the sub areas of the first edition area, the second fluorescent angiographic image photographed at the same angiography timing as the first fluorescent angiographic image is illustrated as an image that is placed automatically, or can be placed, in a second sub area in the second edition area, the second sub area being adjacent in the second direction to the first sub area.
(39) The fundus image observation program of (37) or (38), wherein in the acquisition step, information on the angiography timing of each of the first and second fluorescent angiographic images, together with the image, is acquired, and in the summary edition step, a selection area where the first and second fluorescent angiographic images acquired in the acquisition step are listed, associated with each other according to the angiography timing is displayed simultaneously with the summary edition area, and also at least one of each of the first and second fluorescent angiographic images is selected from the images listed in the selection area to accept a placement operation of placing the first and second fluorescent angiographic images respectively in the sub areas.
(40) The fundus image observation program of any one of (32) to (39), wherein in the summary edition step, a pointer that moves in accordance with an operation on the input interface is displayed in the summary edition area, and also when the pointer is placed on any of the sub areas where the fundus front image is placed, a second pointer indicating the same position on the fundus as the pointer is displayed on the fundus front image in another sub area.
(41) The fundus image observation program of any one of (32) to (40), wherein information indicating either a photographing method or photographing timing of a fundus front image placed in each sub area is displayed in the sub area, associated with the fundus front image.
(42) The fundus image observation program of any one of (32) to (41) causing the computer to further execute a key frame selection step, which is executed before the summary edition step, of displaying, on a key frame selection screen, at least a part of fluorescent angiographic images of a plurality of frames photographed through the fluorescent imaging procedure, and also selecting one or more fluorescent angiographic images on the basis of an examiner's selection operation from the fluorescent angiographic images displayed on the key frame selection screen, wherein in the summary edition step, the key frames selected in the selection step are listed in the selection area.

### BRIEF DESCRIPTION OF THE DRAWINGS

Fig. 1 is a block diagram illustrating an outline of a fundus image observation system according to an example;
Fig. 2 is diagram illustrating the flow of a fluorescent imaging procedure;
Fig. 3 is a diagram illustrating a check screen;
Fig. 4 is a diagram illustrating a frame image list screen;
Fig. 5 is a diagram illustrating parallel display of ICGA photographed images and FA photographed images on the check screen;
Fig. 6 is a diagram illustrating a first modification of the check screen;
Fig. 7 is a diagram illustrating a third modification of the check screen;
Fig. 8 is a diagram illustrating a fourth modification of the check screen;
Fig. 9 is a diagram illustrating a first summary edition screen;
Fig. 10 is a diagram illustrating a second summary edition screen; and
Fig. 11 is a diagram illustrating a third summary edition screen.

### DESCRIPTION OF THE EMBODIMENTS

In the following detailed description, for purpose of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

The present disclosure is described hereinafter on the basis of an embodiment.

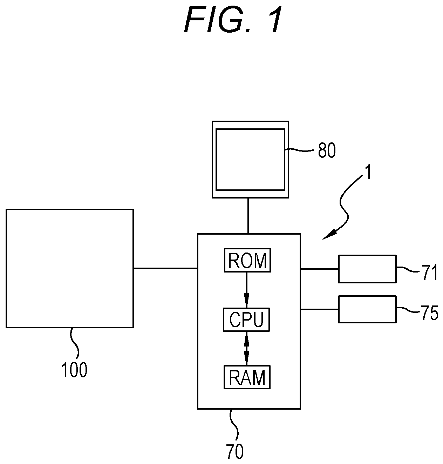

Firstly, a fundus image observation system of the present disclosure is described with reference to Fig. 1. In the embodiment, the fundus image observation system performs fluorescent angiography on the fundus of an examinee's eye. Moreover, a part, which is suitable for observation or diagnosis, of images of a plurality of frames obtained by fluorescent angiography is extracted, and is further displayed. The fundus image observation system includes a computer 1 and a fundus photographing apparatus 100. The computer 1 and the fundus photographing apparatus 100 are connected in a wired or wireless manner. Consequently, the computer 1 and the fundus photographing apparatus 100 can communicate with each other. A fundus image observation program according to the present disclosure is stored in a nonvolatile memory that can be read by a processor of the computer 1. In the embodiment described below, the fundus photographing apparatus 100 and the computer 1 are separate bodies. However, the two apparatuses may be integrated.

### <Fundus Photographing Apparatus>

The fundus photographing apparatus 100 includes at least a photographing optical system (not illustrated). In the photographing optical system, light returned from the fundus of the examinee's eye after irradiating the fundus is received on a photodetector. A fundus front image is formed on the basis of a light receiving signal from the photodetector. The fundus front image is formed by an image processing circuit (for example, an IC and a processor) of the fundus photographing apparatus 100. The image processing circuit does not need to be provided to the fundus photographing apparatus 100. For example, an image processing circuit of the computer 1 may also serve as the image processing circuit of the fundus photographing apparatus 100.

In the embodiment, the fundus photographing apparatus 100 may be capable of photographing a fluorescent angiographic image as a type of fundus front image. Moreover, the fundus photographing apparatus 100 may be capable of photographing a reflection image as another fundus front image. The fluorescent angiographic image is a photographed image by fluorescence emission of a fluorescent contrast agent administered into the blood vessels of the fundus. The reflection image is a photographed image by fundus reflected light of illuminating light used for irradiating the fundus.

The photographing optical system of the fundus photographing apparatus 100 may be capable of photographing a fluorescent angiographic image and a reflection image simultaneously. Simultaneously described here is not limited to completely simultaneously. A little time difference is permitted. The time difference may be set within a range to an extent, for example, that the significant movement of the eye does not occur while two types of fundus front images are photographed.

The fundus photographing apparatus 100 may photograph a fluorescent angiographic image by any of photographing methods such as indocyanine green angiography (ICGA) and fluorescein angiography (FA). Two types of contrast agents may be simultaneously injected into a vein to allow for simultaneous photographing of two types of fluorescent angiographic images. Moreover, the fundus photographing apparatus may be capable of photographing a reflection image by infrared light (hereinafter referred to as the "IR image") or a reflection image by visible light (hereinafter referred to as the "visible light image"). Moreover, the fundus photographing apparatus 100 may be further capable of photographing an autofluorescence image.

Unless otherwise specified, it is assumed in the following description that the fundus photographing apparatus 100 is a scanning laser ophthalmoscope (SLO). However, the fundus photographing apparatus 100 is not necessarily limited to the SLO. For example, the fundus photographing apparatus 100 may be various apparatuses having a function of photographing a fundus front image using fluorescence, such as a fundus camera. The fundus photographing apparatus 100 may be an apparatus that is integral with another ophthalmic apparatus such as an optical coherence tomography (OCT) or a campimeter. Please refer to various known documents (for example, JP-A-2016-59399 of the present applicant) for the detailed optical configuration of the SLO that can photograph a fluorescent angiographic image and a reflection image simultaneously.

### <Computer>

The computer 1 at least acquires various fundus front images photographed by the fundus photographing apparatus 100 and causes a monitor 80 to display the various fundus front images. The computer 1 includes at least a calculation control unit (hereinafter abbreviated to the "control unit") 70. The control unit 70 may be configured including a CPU, a RAM, a ROM, and the like as illustrated by example in Fig. 1. The control unit 70 executes various processes related to a fundus front image, in addition to control over the display of a fundus front image, on the basis of the fundus image observation program.

As illustrated in Fig. 1, the control unit 70 of the computer 1 is connected to a memory 71, an operating unit 75, a monitor 80, the fundus photographing apparatus 100, and the like via a data bus and the like.

In the embodiment, the memory 71 is a nonvolatile storage device. For example, a hard disk or flash memory can be applied as the memory 71. In the embodiment, the fundus image observation program is stored in advance in the memory 71. The memory 71 may be rewritable. In this case, various fundus front images acquired from the fundus photographing apparatus 100 may be stored in the memory 71 as described in the following example. However, the fundus front image may be stored in a memory (not illustrated) different from the memory in which the fundus image observation program is stored.

The operating unit 75 is an input interface of the computer 1. The control unit 70 accepts a signal in accordance with the input of an operation into the operating unit 75. Various devices are conceivable as the operating unit 75. For example, at least one of a touchpad, a mouse, and a keyboard may be used as the operating unit 75. The operating unit 75 may also serve as an input interface of the fundus photographing apparatus 100. A joy stick, a switch, or the like provided to the fundus photographing apparatus 100 may be provided as the input interface (operating unit) of the fundus photographing apparatus 100 to the fundus photographing apparatus 100.

The monitor 80 is used as a display unit (display device) of an image photographed by the fundus photographing apparatus 100 in the embodiment. The monitor 80 may be, for example, a general-purpose monitor or a monitor provided to the apparatus. Instead of the monitor 80, another display device such as a head mount display may be used as the display unit.

### <Description of Operation>

The operation of the fundus image observation system having the above-described configuration is described below.

### <Fluorescent Angiography>

When a contrast agent is injected into a vein of an examinee, the amount of the contrast agent that travels through blood vessels of the fundus increases dramatically a predetermined period of time after the injection, and then reduces gradually. In the early stage of the angiography immediately after the injection, the contrast agent travels through large blood vessels and then small blood vessels. At this point in time, a change in the state of fluorescence emission at the fundus is large and quick. On the other hand, in the middle and later stages of the angiography when time further passed after the injection of the contrast agent, the contrast agent has distributed sufficiently in the vessels. Accordingly, a change in the state of fluorescence emission at the fundus becomes small.

In the early stage of the angiography in the fluorescent imaging procedure from the early stage to the later stage of the angiography, fluorescent angiographic images may be continuously photographed on the basis of a photographing trigger signal (a first photographing trigger signal in the embodiment). In continuous photography, fluorescent angiographic images are continually photographed during a period of at least 10 seconds to several minutes or several tens of minutes after the output of the photographing trigger signal. In other words, during this period, light is projected onto the fundus and the light returned from the fundus is received on the photodetector. As a result of continuous photography, a photographed image group is acquired by the fundus photographing apparatus 100.

The photographed image group includes fluorescent angiographic images (fundus front images with fluorescence) of a plurality of frames continuously photographed at regular intervals after the output timing of the photographing trigger signal (the first photographing trigger signal in the embodiment). In the SLO, the fundus is periodically scanned with light within a predetermined area, and accordingly, it is possible to obtain a photographed image of one frame per scan (that is, in one cycle) with the light in the predetermined area. The photographed image group may include a fluorescent angiographic image obtained by a fundus scan in every cycle during the photographing period. Alternatively, the photographed image group may include a fluorescent angiographic image obtained by a fundus scan in every other cycle (or may be every two cycles, every three cycles, ..., every n cycles). Photographed images are obtained at least several frames per second by the fundus photographing apparatus 100.

Each fluorescent angiographic image included in the photographed image group may be referred to below as the "frame image" for the sake of convenience.

Moreover, in the middle and later stages of the angiography in the fluorescent imaging procedure, a fluorescent angiographic image may be singly photographed on the basis of the output of a photographing trigger signal (a second photographing trigger signal in the embodiment). In this case, a still image of a fluorescent angiographic image is singly photographed immediately after the output of the photographing trigger signal. "Single photography" here indicates that a predetermined number of fluorescent angiographic images - at least one fluorescent angiographic image - is obtained triggered by the output of the photographing trigger signal. In "single photography," photographing is performed in a shorter time than continuous photography. It is preferable that the number of fluorescent angiographic images obtained per photographing trigger signal be limited to approximately ten at the maximum. The fluorescent angiographic image obtained as a result of "single photography" is referred to below as the "still angiographic image" for the sake of convenience. The still angiographic image may be an image having more pixels (for example, a higher resolution) than a frame image obtained by continuous angiography.

Fluorescent angiography is not necessarily limited to these techniques described. A still angiographic image may be photographed in the early stage of the angiography, and continuous angiography may be performed in the later stage of the angiography.

A part or all of a plurality of fluorescent angiographic images acquired as a result of the fluorescent imaging procedure may be added images. In both FA and ICGA, fluorescence from the fundus is feeble, which is especially remarkable in the early and later stages of the angiography. Hence, it may be configured, for example, that fluorescent angiographic images of a plurality of continuous frames are acquired, and added (may be averaged or averaged with a weight or the like) to obtain an excellent fluorescent angiographic image.

The fundus photographing apparatus 100 photographs a reflection image simultaneously with each fluorescent angiographic image. The reflection image can be used to associate the positional relationship of each fluorescent angiographic image. The details of the reflection image are described below.

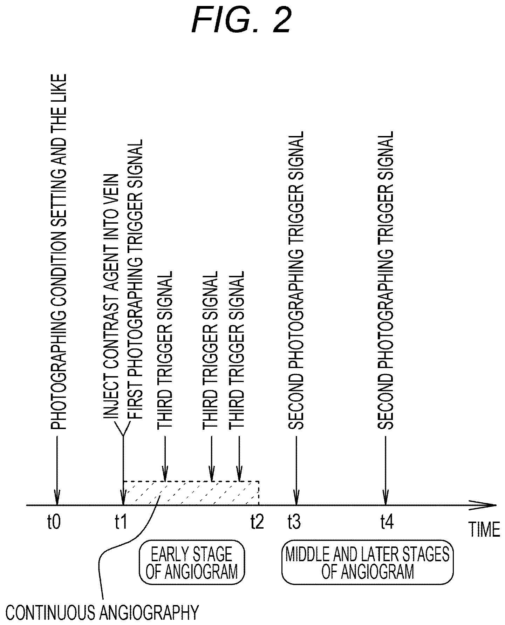

Next, specific examples of the apparatus operation in fluorescent angiography are described with reference to Fig. 2.

### [t0: Alignment and Setting of Photographing Conditions]

Firstly, an examiner adjusts the alignment of the apparatus with an examinee's eye, and also sets photographing conditions. The photographing conditions set in this case include at least a condition on the wavelength of light irradiating the fundus as excitation light. In a case of, for example, FA, blue or green light is set as excitation light. In a case of ICGA, light in an infrared range is set as excitation light. When FA and ICGA are performed simultaneously, two types of wavelength ranges corresponding to FA and ICGA are set as excitation light. Furthermore, in the embodiment, light in a second infrared range is further set as light irradiating the fundus. The light in the second infrared range serves as both light for observing the fundus and light for obtaining a reflection image. The second infrared range and the infrared range for the excitation of ICG belong to wavelength ranges different from each other.

### [t1: Start of Fluorescent Angiography]

When the alignment and the setting of the photographing conditions are complete, the examiner injects a contrast agent into a vein of the examinee. Moreover, simultaneously (or immediately afterwards), an operation for starting the fluorescent imaging procedure is input into the input interface of the fundus photographing apparatus 100. As a result, fluorescent angiography is started in the fundus photographing apparatus 100.

Firstly, an angiography timer of the fundus photographing apparatus 100 is activated. The angiography timer is a time measurement device that measures elapsed time from the start of the angiography. In other words, the angiography timing is managed by the angiography timer. Each of the fundus front images (fluorescent angiographic images and reflection images) photographed in the fluorescent imaging procedure is associated with the measurement time of the angiography timer at each photographing timing, and stored in the memory.

Through the fluorescent imaging procedure, the fundus may be continually irradiated with the excitation light (and further scanned in the SLO), and then fundus images based on fluorescence from the fundus resulting from the irradiation with the excitation light may be sequentially acquired by the fundus photographing apparatus 100. The sequentially acquired images may be displayed as an observation image (real-time moving image) on a monitor (for example, the monitor 80). Consequently, the photographing area and the displacement of fixation can be checked from the reflection image. The examiner can perform a trigger operation at an appropriate timing while checking the observation image based on fluorescence.

### [t1 to t2: Early Stage of Angiography]

In the embodiment, continuous angiography is started at the timing of start of the fluorescent imaging procedure. In other words, the photographing trigger signal of continuous angiography is output on the basis of an operation of starting continuous angiography. As a result of continuous angiography, a photographed image group including fluorescent angiographic images of a plurality of frames is stored in the memory. In the embodiment, reflection images of a plurality of frames photographed simultaneously with the corresponding fluorescent angiographic images are stored in the memory. Continuous angiography may be terminated automatically a fixed period of time after the start, may be terminated manually on the basis of a predetermined operation on the input interface of the fundus photographing apparatus 100, or may be terminated when another termination condition is satisfied.

In the embodiment, frame images (fluorescent angiographic images) acquired sequentially are displayed as a real time moving image on the monitor 80. In addition, reflection images photographed together with the corresponding frame images may be displayed as a moving image. The photographing area and the displacement of fixation can be checked from the reflection images.

A frame image obtained at an output timing of a trigger signal (third trigger signal) emitted during continuous angiography may be selected as a key frame (or candidate therefor). In this case, information for specifying a key frame (or candidate therefor) from the photographed image group obtained as a result of continuous angiography (key frame specification information) is stored in the memory (a second acquisition step).

For example, the trigger signal for selecting a key frame during continuous angiography may be output on the basis of an operation on the input interface of the fundus photographing apparatus 100. In the embodiment, the examiner performs the above-described operation (this operation is referred to below as the "trigger operation") on the input interface at a timing when recognizing a desired contrast medium appearance in the moving image to input the third trigger signal into the fundus photographing apparatus 100.

In the embodiment, a "key frame" is an image illustrating a desired contrast medium appearance among a plurality of frame images obtained as a result of continuous angiography. For example, a frame image formed within several seconds during which the contrast agent travels through choroidal neovascularization (CNV), the frame image being useful for diagnosis, may be selected as one of key frames. Key frames of a plurality of frames can be selected for one continuous angiography session. In the embodiment, a new key frame (or candidate therefor) is additionally selected on every trigger operation during continuous angiography.

Various kinds of information can be used for the "key frame specification information." For example, it may be photographing time information indicating the time when a key frame (or candidate therefor) is photographed. The photographing time information may indicate the date and time of acquisition of a key frame (or candidate therefor), or the measurement time of the angiography timer at the time of acquisition of a key frame (or candidate therefor). Moreover, when frame images are serial numbered, serial number information assigned to the key frame (or candidate therefor) can be used as the "key frame specification information." In addition, information other than those illustrated here by example can be used as the key frame specification information

An example where a candidate for a key frame is selected on the basis of the trigger operation during continuous photography is mainly described below. It can be determined anew whether or not the selected candidate is selected as a key frame in the end after the completion of the fluorescent imaging procedure (the details are described below).

The above-described trigger operation may be the same as the operation at the start of the photographing of still angiographic images. For example, the push of a release button (a type of input interface of the fundus photographing apparatus 100) may be the trigger operation.

As described above, continuous angiography is performed in the early stage of the angiography.

### [t2 to t3: Middle and Later Stages of Angiography]

Next, a still angiographic image is photographed after approximately several to several tens of minutes as appropriate. At the time of photographing, the fundus photographing apparatus 100 acquires the observation image based on the excitation light or the light in the infrared range and causes the monitor 80 to display the acquired image. The photographing operation is performed (for example, the release button is pressed) at any timing in the middle and later stages of the angiography while the contrast medium appearance or photographing area is checked using the observation image. The photographing trigger signal (second photographing trigger signal) is input into the fundus photographing apparatus 100 on the basis of the photographing operation. Consequently, the fundus photographing apparatus 100 acquires a still angiographic image. At this point in time, the light for photographing a reflection image, together with the excitation light, is projected onto the fundus to obtain a reflection image simultaneously with the still angiographic image. Consequently, in the middle and later stages of the angiography, a still angiographic image and a reflection image are acquired simultaneously on every photographing operation and are stored in the memory.

### <Acquisition of Photographing Result>

Fluorescent angiographic images and reflection images, which are photographed by the fundus photographing apparatus 100 as described above, are stored in a memory (the memory 71 here) that is accessible from the processor of the computer 1. At this point in time, image group data including fluorescent angiographic images of a plurality of frames (and also reflection images in the embodiment) obtained through the fluorescent imaging procedure is stored in the memory 71 (a first acquisition step). For example, one file or folder may be generated per one set of image group data in the memory 71. The file or folder can contain data of each image obtained through the fluorescent imaging procedure. In the embodiment, a description is given assuming one set of image group data to be generated through each fluorescent imaging procedure. However, one set of image group data may be generated every photographing day.

As described above, the information indicating the measurement time of the angiography timer at the time when each fluorescent angiographic image and reflection image is photographed is further stored in the memory 71 in the embodiment.

Moreover, the third trigger signal for selecting a key frame (or a candidate therefor) may be output halfway through continuous angiography, and key frame specification information may be further generated on the basis of the output. In this case, the key frame specification information, together with data of a photographed image group obtained by continuous angiography, is stored in the memory 71.

### <Photographing Result Check Screen>

The control unit 70 of the computer 1 generates a check screen on which fluorescent angiographic images of an examinee's eye are displayed in list form (in the embodiment, the check screen also serves as a key frame selection screen) per fluorescent imaging procedure, or every photographing day. The control unit 70 of the computer 1 causes the monitor 80 to display the check screen (a display step; a list display step).

Through the fluorescent imaging procedure, a fluorescent angiographic image photographed at any given angiography timing can be selected on the check screen from image group data including fluorescent angiographic images of a plurality of frames obtained per photographing trigger signal. Furthermore, the selected image can be displayed on the check screen. In the embodiment, an operation of selecting an angiography timing is accepted via the operating unit 75. A fluorescent angiographic image corresponding to the angiography timing is selected as an image displayed on the check screen. If a plurality of fluorescent angiographic images is selected, each of the selected images may be displayed in a line, or switched and displayed in one area, on the check screen. In this case, still angiographic images and frame images included in a photographed image group are arranged chronologically in a mixed state, or switched chronologically. In other words, an angiography timing of each frame image and an angiography timing of each still angiographic image are regarded as a time series, and an image at any given angiography timing in the time series is selected and then displayed.

A widget (control) for accepting the operation of selecting an angiography timing may be provided on the check screen. For example, a slider corresponding to a time series in the fluorescent imaging procedure may be provided as the widget. In this case, an image at an angiography timing corresponding to the position of a lever of the slider is displayed on the check screen.

The operation of selecting an angiography timing is not necessarily required to be an operation via the slider. For example, a widget other than the slider may be displayed on the monitor. Moreover, the operation of selecting an angiography timing does not need to be a selection operation via the widget on the monitor 80, and can use various operations such as an operation on a specific key (for example, keys of "↑", "↓", "←", and "→") on a keyboard and a mouse wheel operation.

Moreover, each of the still angiographic images and the frame images, which are displayed in list form on the check screen, is displayed, associated with time information indicating an angiography timing thereof. Reference to the time information facilitates finding an image illustrating a contrast medium appearance desired by the examiner from many fluorescent angiographic images.

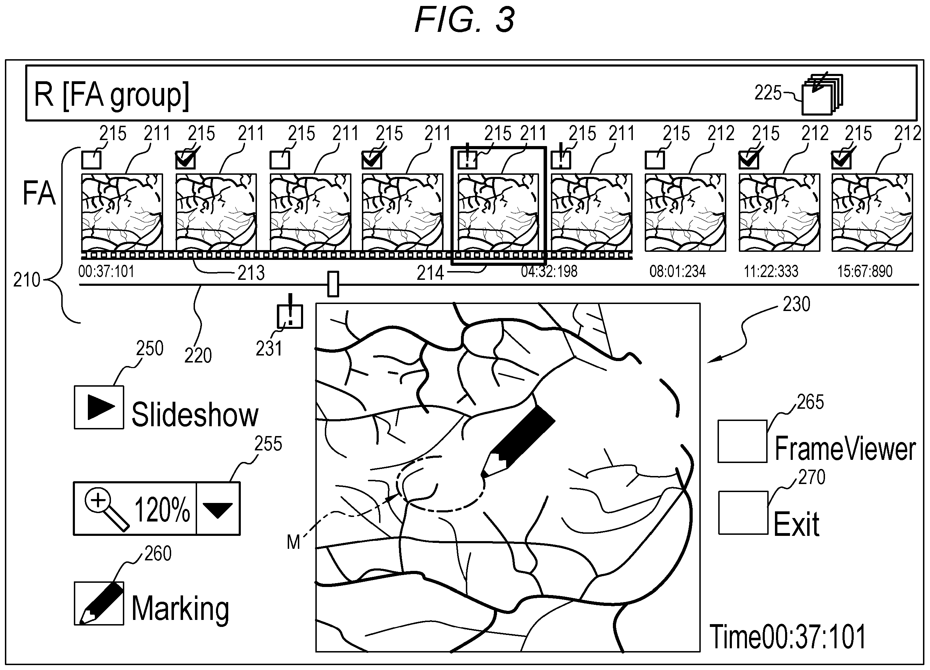

Fig. 3 illustrates a photographing result of FA as one of examples of the check screen (also serving as the key frame selection screen). In Fig. 3, a thumbnail display area 210 is provided in a predetermined position in the upper part of the screen. The still angiographic images and the frame images, which are acquired through the fluorescent imaging procedure, are arranged chronologically and displayed in the thumbnail display area 210, using their respective thumbnails 211 and 212. In Fig. 3, the reference numeral 211 indicates a thumbnail of a frame image, and the reference numeral 212 indicates a thumbnail of a still angiographic image.

The angiography timings are displayed with the time information near (below, in Fig. 3) the thumbnails 211 and 212 on the check screen of Fig. 3.

A part of the frame images is extracted from the plurality of frame images included in the photographed image group, and displayed (as the thumbnails 211) in the thumbnail display area 210. The number of frame images displayed in the thumbnail display area 210 may be predetermined. For example, a fixed number of frame images may be displayed per photographed image group. Alternatively, the number of frame images per photographed image group may correspond to the photographing time of continuous angiography, or the number of frame images included in a photographed image group. Moreover, the number of frame images displayed in the thumbnail display area 210 may be determined on the basis of the examiner's selection operation. As a specific example in this case, each of frame images selected as a candidate for a key frame (may be "as a key frame") during continuous angiography (refer to the period from [t1 to t2] in Fig. 2) may be displayed as the thumbnail 211 in the thumbnail display area 210. Furthermore, in this case, if the trigger operation for selecting a candidate for a key frame is not performed during continuous angiography, a predetermined number of frame images may be extracted automatically, and displayed as the thumbnails 211 in the thumbnail display area 210. Moreover, if continuous angiography is performed a plurality of times in the fluorescent imaging procedure, at least one frame image may be extracted in each continuous angiography session (per first photographing trigger signal), and displayed in the thumbnail display area 210. Moreover, the number of frame images extracted and displayed in the thumbnail display area 210 may be changeable.

The control unit 70 associates an identification graphic for distinguishing a still angiographic image and a frame image with at least one of a still angiographic image and a frame image, which are displayed on the check screen, and causes the monitor 80 to display the graphic. For example, among the thumbnails 211 and 212, only the thumbnail 211 is displayed with a graphic 213 in Fig. 3. In Fig. 3, the graphic 213 is customarily used in each field as a graphic mimicking sprocket holes of a photographic film to suggest thumbnails of a moving image. Consequently, the examiner can intuitively grasp the thumbnail 211 as a thumbnail of a frame image (that is, an image extracted from a photographed image group including many frames).

Moreover, a slider 220 is placed near (below, in Fig. 3) the thumbnail display area 210 on the check screen of Fig. 3. The slider 220 is a widget for accepting the operation of selecting an angiography timing in the embodiment. When a lever of the slider 220 is moved by, for example, a drag operation using a pointing device (a type of operating unit 75), the thumbnail 211 or 212 tied to the position of the lever is displayed.

A detailed display area 230 is provided at a position different from the thumbnail display area 210 on the check screen of Fig. 3. A fluorescent angiographic image of a larger size or with a higher number of pixels than a thumbnail is displayed in the detailed display area 230. The examiner can check the contrast medium appearance at a desired angiography timing in detail by checking the detailed display area 230.

A fluorescent angiographic image displayed in the detailed display area 230 may correspond to one of the thumbnails 211 and 212 (on the basis of the same image data). In this case, a thumbnail targeted for detailed display may be selected from the thumbnails 211 and 212, on the basis of the examiner's selection operation. Examples of the selection operation include click or tap of a desired thumbnail 211 or 212 via the pointing device, and then drag-and-drop to the detailed display area 230. However, the selection operation may be any other operation method. As illustrated in Fig. 3, a graphic 214 for distinguishing the thumbnail 211 or 212 that has been selected (is being displayed in detail) from other thumbnails may be displayed for association.

### <Alignment between Images>

An alignment button 225 is placed on the check screen of Fig. 3. The selection of the alignment button 225 causes fluorescent angiographic images displayed on the check screen to be aligned (a displacement correction step). As a result, displacements of photographing positions of fluorescent angiographic images displayed are corrected with reference to a certain fluorescent angiographic image. Each fluorescent angiographic image has a difference in contrast medium appearance. Accordingly, it may be difficult to align the images. Hence, in the embodiment, for example, a reflection image photographed simultaneously with a fluorescent angiographic image is used to adjust alignment. Please refer to, for example, JP-A-2016-59400 for the details of the alignment.

In the embodiment, in terms of alignment, any fluorescent angiographic image (or a reflection image photographed simultaneously with the fluorescent angiographic image) may be used as a reference for alignment. If key frames have already been selected, for example, the key frame having the earliest angiography timing may be used as a reference for alignment. Moreover, a frame image or still angiographic image selected as a detailed display target may be used as a reference for alignment.

In this case, an image that is displayed in the detailed display area 230 when the alignment button 225 is selected serves as a reference for alignment.

When alignment is adjusted, images displayed in the thumbnail display area 210 and the detailed display area 230 are displayed, aligned with the fluorescent angiographic image serving as the reference. Hence, it is easy to compare the fluorescent angiographic images on the check screen.

### <Highlight of Region of Interest in Fluorescent Angiographic image>

In the embodiment, a region of interest may be highlighted in a fluorescent angiographic image displayed in the detailed display area 230 (a highlight step). For example, a region of interest may be highlighted by enlarged display, or by superimposed display in which a marker is superimposed on the region of interest or its vicinity. The highlight modes are not limited to the enlarged display and the marker superimposed display. Various other highlight modes can be applied. For example, a region of interest and other parts may be displayed in different modes to apply a highlight.

A highlight is applied on the basis of at least information for identifying the position of a region of interest (or its vicinity) (hereinafter referred to as the "region-of-interest position information"). The region-of-interest position information is information that identifies a region of interest in any of a plurality of fluorescent angiographic images obtained through the fluorescent imaging procedure. The region-of-interest position information may be, for example, position information on a fluorescent angiographic image or a reflection image photographed simultaneously with the fluorescent angiographic image. The position of a region of interest may be any position selected by the examiner on a fluorescent angiographic image or reflection image displayed on the monitor 80. Moreover, the position of a region of interest may be a predetermined position on a fluorescent angiographic image or reflection image, or a position of a lesioned part or the like detected from a fluorescent angiographic image or reflection image. A case in which a region of interest is set on the basis of the input of the examiner's operation is described below.

A part of a fluorescent angiographic image is enlarged and displayed in the detailed display area 230 to apply a first highlight. In this case, the highlight may be able to be enlarged with any given position on the fluorescent angiographic image as the center. The position to be centered may be input, for example, via the operating unit 75. In Fig. 3, if, for example, a widget (control) 255 is selected, options for a display scale are displayed in drop down form. A desired display scale can be selected from the options. The center position at the time of enlargement is then selected in the detailed display area 230 to display, in the detailed display area 230, the fluorescent angiographic image that has been enlarged at the selected display scale with the position as the center. Naturally, a technique for selecting a position for enlargement is not limited to the above-described technique. In this manner, a given position on a fluorescent angiographic image is enlarged and displayed to make it easy for the examiner to check a contrast medium appearance in a fine region such as the travel of the contrast agent through capillaries and leakage of the contrast agent from new blood vessels. In this case, information indicating the enlargement center position on the fluorescent angiographic image displayed in the detailed display area 230 is stored as the region-of-interest position information in the memory 71. Moreover, the magnification rate is associated together and stored in the memory 71.

In the embodiment, a marker M may be able to be superimposed on the fluorescent angiographic image displayed in the detailed display area 230. For example, the marker M may be superimposed at a position designated on the basis of the input of an operation on the operating unit 75 on the fluorescent angiographic image. A graphic of the marker M may be predetermined, or drawn freehand in accordance with an operation on the operating unit 75. For example, a free-form curve is a typical example of a graphic drawn freehand. In Fig. 3, for example, when a button 260 is selected, the marker M can be drawn (placed) in the detailed display area 230. The examiner moves a pointer over a region of interest, or the like, and performs the operation of placing the maker M, and accordingly superimposes the marker M at the position of the pointer. In this case, information on the position of the marker M associated with the fluorescent angiographic image displayed in the detailed display area 230 is stored as the region-of-interest position information in the memory 71.

### <Switching of Image to Be Displayed in Detailed Display Area>

In terms of a fluorescent angiographic image displayed in the detailed display area 230 among a plurality of fluorescent angiographic images acquired through the fluorescent imaging procedure, it is possible to switch from a certain image (hereinafter referred to as the "first image") to another image (hereinafter referred to as the "second image"). Each of the first and second images may be freely selected from the plurality of fluorescent angiographic images acquired through the fluorescent imaging procedure, on the basis of the examiner's selection operation. Moreover, the first and second images may be selected on the basis of predetermined stipulated rules. The stipulated rules here may be, for example, rules related to the photographing order of the fluorescent angiographic images. For example, the plurality of fluorescent angiographic images acquired through the fluorescent imaging procedure may be sequentially switched and displayed in photographing order at predetermined time intervals as in slide show display. In the example of Fig. 3, the above-described slide show display is performed on the basis of an operation of selecting a button 250. Moreover, in the example of Fig. 3, for example, any thumbnail that is different from a thumbnail being displayed in detail (that is, a thumbnail associated by the graphic 214) can be selected as a new detailed display target from the thumbnails 211 and 212, on the basis of the examiner's selection operation.

### [Reproduction of Highlight]

When an image displayed in the detailed display area 230 is switched from the first image to the second image, if there is a highlight on a region of interest on the first image, the highlight of the region of interest on the first image may also be reproduced on the second image. In this case, the same region of interest as that of the first image is highlighted on the second image on the basis of the region-of-interest position information of the first image. More specifically, a first reflection image photographed simultaneously with the first image and a second reflection image photographed simultaneously with the second image are aligned with each other. The first image and the first reflection image, and the second image and the second reflection image have the same photographing area. Hence, as a result of the alignment, the position of the region of interest on the first reflection image can be identified on the second reflection image. As a result, the examiner can easily grasp a change in the region of interest over time.

### <Selection of Key Frames: Check Screen>

In the embodiment, one or more frames illustrating a desired contrast medium appearance are selected and set as key frames from a plurality of fluorescent angiographic images obtained through the fluorescent imaging procedure. As a result, the key frame specification information for specifying key frames from a photographed image group is stored in the memory 71. Key frames extracted from image group data related to the fluorescent imaging procedure can be displayed in list form on the basis of the image group data and the key frame specification information corresponding to the image group data. Such a list may be displayed according to the fluorescent imaging procedure, or the photographing date. In this manner, fluorescent angiographic images illustrating a desired contrast medium appearance are extracted from fluorescent angiographic images of a plurality of frames obtained through the fluorescent imaging procedure, and displayed in list form. Hence, it is easy for the examiner to excellently check angiographic images that become points for observation and diagnosis through the fluorescent imaging procedure.

In the embodiment, the key frames may be selected and set on the basis of the examiner's selection operation. It is generally conceivable that approximately several (for example, less than 10) fluorescent angiographic images illustrate a characteristic contrast medium appearance at each angiography timing in a fluorescent imaging procedure. Hence, it is assumed in the embodiment that approximately several key frames are selected in each fluorescent imaging procedure.

However, it is conceivable that many fluorescent angiographic images are photographed in each fluorescent imaging procedure. In a case of, for example, continuous photography, several tens or more frames are generated only for approximately several seconds. Moreover, it is also conceivable to allow for the number of photography sessions considering an error in photography.

Hence, it is considered that presenting all frames obtained through the fluorescent imaging procedure to the examiner, and causing the examiner to select key frames from all the frames easily causes a burden on the examiner.

Hence, in the embodiment, a part of a plurality of fluorescent angiographic images obtained through the fluorescent imaging procedure is extracted and displayed on the check screen. Images displayed can be then selected as key frames. In other words, a part of a plurality of fluorescent angiographic images is set as selection targets in the operation of selecting key frames (a reselection step).

As described above, especially a photographed image group generated as a result of continuous photography includes many frames. Hence, it is preferable to limit at least the number of frame images displayed on the check screen among the photographed image group. Hence, among the frame images included in the photographed image group, frame images extracted at predetermined frame intervals, or in a predetermined number, may be displayed on the check screen. Moreover, a frame image acquired at a timing when the trigger operation is performed during continuous angiography may be displayed as a candidate for the key frame on the check screen.

In the example of Fig. 3, candidates for key frames and key frames selected from the candidates are displayed as the thumbnails 211 in the thumbnail display area 210. Check boxes 215 are placed, associated respectively with the thumbnails 211. Selected state information indicating whether the thumbnail 211 is a key frame, a candidate therefor, or neither is presented in the check box 215. A check mark (√) is put in the check boxes 215 of thumbnails of key frames on the screen of Fig. 3. Moreover, the check boxes 215 of thumbnails of candidates for key frames are blank or contain an exclamation mark. The exclamation mark is put in the check boxes 215 of candidates for key frames selected on the basis of the trigger operation during continuous angiography. The check boxes 215 of the other candidates are blank. In Fig. 3, key frames can be selected or deselected via such a check screen on the basis of the examiner's operation. A cursor is placed on the check box 215 that is blank or contains the exclamation mark to click or tap the check box 215. Accordingly, a frame image corresponding to the check box 215 is selected as a key frame. Moreover, the selected state information in the clicked check box 215 is switched to the check mark. On the other hand, a key frame is deselected by clicking or tapping the check-marked check box 215. In this case, the check box 215 returns to a state before the check mark has been added.

In the thumbnail display area 210, a graphic for distinguishing a candidate for a key frame selected on the basis of the trigger operation during continuous angiography from other frames (here, the exclamation mark in the check box 215) is assigned to the candidate. Hence, an image illustrating a desired contrast medium appearance can be selected as a key frame with a reference to an image obtained on the basis of the trigger operation.

### [Determination of Failure in Photography]

An image determined to be a failure in photography is excluded from the key frame selection targets (a determination step). For example, the thumbnail display area 210 of Fig. 3 may be set in such a manner as not to display a thumbnail of the image that has been determined to be a failure in photography. Examples of fluorescence angiographies that have failed in photography include an image whose photographing area has been displaced largely from an intended photographing area due to the movement of the eye and an image including a shadow over a part or whole of the image due to a blink. The control unit 70 may be set in such a manner as to determine whether or not each frame is an image that has failed in photography, and prevent the image determined to have failed in photography from becoming a candidate for a key frame. If a fluorescent angiographic image is a failure in photography, a reflection image photographed simultaneously is also a failure in photography. Hence, the above-described determination may be made on the basis of the reflection image. A change in contrast medium appearance is large and quick especially in the early stage of the angiography. Hence, it is conceivable that it is difficult to determine a failure in photography from a fluorescent angiographic image itself. However, the photographing conditions of a reflection image are constant. Accordingly, a failure in photography can be easily determined.

An image that needs to be set as a key frame or a candidate therefor on the basis of the trigger operation during continuous angiography may be determined as an image that has failed in photography. In this case, an image whose photographing timing is the closest among the image and images that have not failed in photography may be selected as a key frame or a candidate therefor.

Moreover, a check box 231 is also associated with a fluorescent angiographic image displayed in detail in the detailed display area 230. The check box 231 is tied to the check box 215 of the corresponding thumbnail 211. A key frame can also be selected or deselected on the basis of an operation on the check box 215. Consequently, it is easy to select a key frame while checking the details of a fluorescent angiographic image selected as the key frame or as a candidate for the key frame.

The selection of the key frame allows for the formation of the key frame specification information for specifying a key frame from data of a photographed image group to store the key frame specification information in the memory 71. If the key frame specification information has already been generated on the basis of the trigger operation during continuous angiography, the information may be overwritten. A button 270 is selected to close the check screen illustrated in Fig. 3. At this point in time, the key frame specification information related to the fluorescent imaging procedure is confirmed.

It is conceivable that still angiographic images more than a minimum number are photographed with allowance through the fluorescent imaging procedure, considering possibilities of an error in photography due to the movement of the eye, and the like. Hence, in the embodiment, a key frame of a still angiographic image can also be selected on the check screen of Fig. 3 by the operation of checking the check box 215 as in the case of a frame image. The key frame specification information may also include information for specifying a key frame of a still angiographic image. Moreover, among still angiographic images, only data of images with the check marks in the check boxes 215 may be left as it is in image group data, and the remaining data may be discarded.

The frame intervals between frame images displayed by being extracted from a photographed image group, or the number of frame images extracted from each photographed image group, may be changeable on the check screen of Fig. 3 in accordance with an operation on the operating unit 75.

In the embodiment, after the key frame specification information is confirmed, the key frames are not displayed on the basis of an image file obtained by extracting only the key frames. Instead, the key frames are displayed on the basis of image group data acquired through the fluorescent imaging procedure and the key frame specification information. Hence, the key frames can be collectively managed without being separated from data of the other fluorescent angiographic images (mainly continuous photography) acquired through the fluorescent imaging procedure. Hence, for example, if it is desired to check frames before and after the key frame displayed on the list display screen, the frames can be easily checked.

### <Selection of Key Frames: Frame Image List Screen>

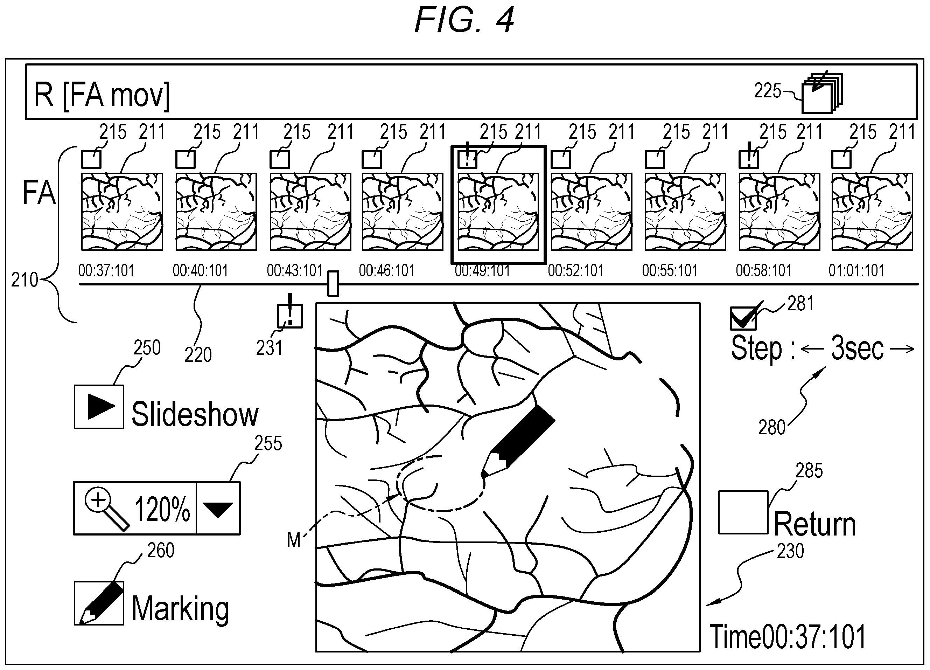

When a button 265 is selected by the pointing device or the like, the check screen illustrated in Fig. 3 is switched to a frame image list screen illustrated in Fig. 4. The screen of Fig. 4 is used to view more frame images included in a photographed image group than those on the screen of Fig. 3, and is also used to select an image illustrating a desired contrast medium appearance as a key frame from the frame images viewed.

The thumbnail display area 210, the slider 220, the detailed display area 230, the widget 255, the button 260, the check boxes 215, and the like are also placed on the screen of Fig. 4 as in Fig. 3. Unless otherwise specified, elements to which the same reference numerals as those of Fig. 3 are assigned have similar uses and functions to the description of Fig. 3 in Fig. 4. Accordingly, the detailed description of the elements is omitted.

Only frame images of fluorescent angiographic images of a plurality of frames photographed through the fluorescent imaging procedure are selectively displayed in the thumbnail display area 210 on the screen of Fig. 4. The frame images are arranged and placed chronologically as the thumbnails 211 in the thumbnail display area 210.

(The thumbnails 211 of) frame images extracted at predetermined time intervals (however, the interval is longer than a photographing frame period of continuous angiography) may be arranged chronologically in the thumbnail display area 210 on the screen of Fig. 4.

In this case, the intervals for extracting frame images arranged in the thumbnail display area 210 may be changeable. For example, frame images for which the extraction interval has been changed may be arranged in the thumbnail display area 210 on the basis of an operation of selecting the extraction interval. The cursor is placed on a widget 280 on the screen of Fig. 4 to click or tap the widget 280. Accordingly, predetermined options for the extraction interval are displayed. Any of the options is selected on the basis of the selection operation via the operating unit 75. Accordingly, frame images extracted at the selected extraction intervals are displayed in the thumbnail display area 210. It is preferable that at least an interval of several seconds within the range of a one-second interval to a ten-second interval be able to be selected as the extraction interval. In the early stage of the angiography, it may become important to observe a contrast medium appearance presented for approximately several seconds. Hence, when frame images are arranged at intervals of approximately several seconds, it is easy for the examiner to find frame images illustrating a desired contrast medium appearance. Naturally, a selectable extraction interval may be less than one second, or greater than 10 seconds.

In Fig. 4, a check box 281 provided near the widget 280 is checked (that is, the cursor is placed on the blank check box 281 to click or tap the check box 281). Accordingly, frame images are extracted and displayed (the number of frame images is reduced for display) as describe above. On the other hand, when the check box 281 is blank, all the frame images are arranged chronologically and displayed in the thumbnail display area 210. For example, the angiography time presenting a desired contrast medium appearance is roughly grasped by using the extracted display, and then the state is changed to a state where all the frame images are arranged. Accordingly, suitable key frames can be selected soon.

When the extracted display is presented, frame images selected as candidates for key frames on the basis of the trigger operation during continuous angiography, in addition to the frame images extracted at the predetermined time intervals, may be arranged in the thumbnail display area 210. In this case, the examiner can distinguish between the frame images obtained on the basis of the trigger operation and the other frame images by the presence or absence of the exclamation marks in the check boxes 215. As a result, a frame image illustrating a desired contrast medium appearance can be selected as a key frame with reference to the frame images obtained on the basis of the trigger operation.

A button 285 is selected on the screen of Fig. 4 to switch to the screen of Fig. 3.

When a frame image included in a photographed image group is displayed in the detailed display area 230 on the check screen of Fig. 3 or 4, the trigger operation may be performed via the operating unit 75 while frame images included in the photographed image group are switched and displayed in photographing order, and accordingly a frame image displayed in the detailed display area 230 may be selected as a key frame at the timing when the trigger operation is performed (the output timing of the trigger signal).

### <Simultaneous Check Screen>

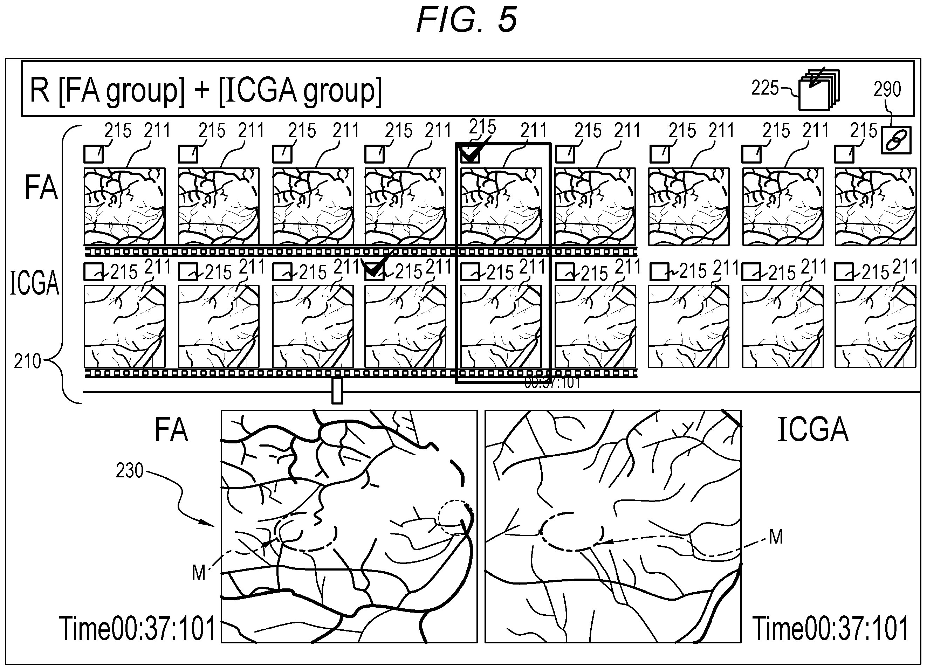

Two or more types of fluorescent angiography may be carried out on an examinee's eye in a day. When the computer 1 has acquired the photographing results, instead of the check screen of Fig. 3, a check screen illustrated in Fig. 5 may be displayed. Fluorescent angiographic images obtained by FA and fluorescent angiographic images obtained by ICGA are displayed separately in list form in the thumbnail display area 210 on the screen of Fig. 5. Thumbnails 311 of the fluorescent angiographic images obtained by FA and thumbnails 312 of the fluorescent angiographic images obtained by ICGA are displayed vertically in lines in the thumbnail display area 210. The thumbnails 311 and 312 may be arranged, associated according to the angiography time. For example, in a case of the screen of Fig. 5, thumbnails having the same angiography timing are placed in such a manner as to be vertically adjacent.

As in Fig. 3, the thumbnail display area 210, the slider 220, the detailed display area 230, the widget 255, the button 260, the check boxes 215, and the like are also placed on the screen of Fig. 5. Unless otherwise specified, elements to which the same reference numerals as those of Fig. 3 are assigned have similar uses and functions to the description of Fig. 3 in Fig. 5. Accordingly, the detailed description of the elements is omitted.

The two types of fluorescent angiographic images of FA and ICGA are simultaneously displayed in detail in the detailed display area 230 in Fig. 5. A display area 230a for FA and a display area 230b for ICGA are set in the detailed display area 230. Hence, a fluorescent angiographic image obtained by each of FA and ICGA can be checked excellently.

Whenever a button 290 is selected, the turning-on and -off of a simultaneous link setting is switched. If the simultaneous link setting is off, the detailed display target can be selected from each of the thumbnails 311 and 312 of FA and ICGA. On the other hand, if the simultaneous link setting is on, any one of the thumbnails 311 and 312 of FA and ICGA can be selected as the detailed display target. A thumbnail having the same elapsed time from the start of the angiography as the selected thumbnail is selected automatically as the detailed display target from thumbnails of fluorescent angiography different from the selected thumbnail. Consequently, the burden of an operation on the examiner is reduced upon the detailed display.

The timing when a fluorescent angiographic image useful for observation and diagnosis is obtained varies depending on the type of angiography. Hence, a key frame can be selected independently on the screen of fig. 5 in each type of fluorescent angiography.

When there is a highlight on a region of interest on a fluorescent angiographic image of one of FA and ICGA displayed in the detailed display area 230, the same region as the region of interest may also be highlighted automatically as the region of interest on a fluorescent angiographic image of the other. For example, as in the method described under the heading of [Reproduction of Highlight], the same region as the region of interest on the fluorescent angiographic image of one of FA and ICGA may be identified on the fluorescent angiographic image of the other on the basis of a result of the alignment between a reflection image photographed simultaneously with the fluorescent angiographic image of one of FA and ICGA, and a reflection image photographed simultaneously with the fluorescent angiographic image of the other.

### <Modifications of Check Screen>

### [First Modification]

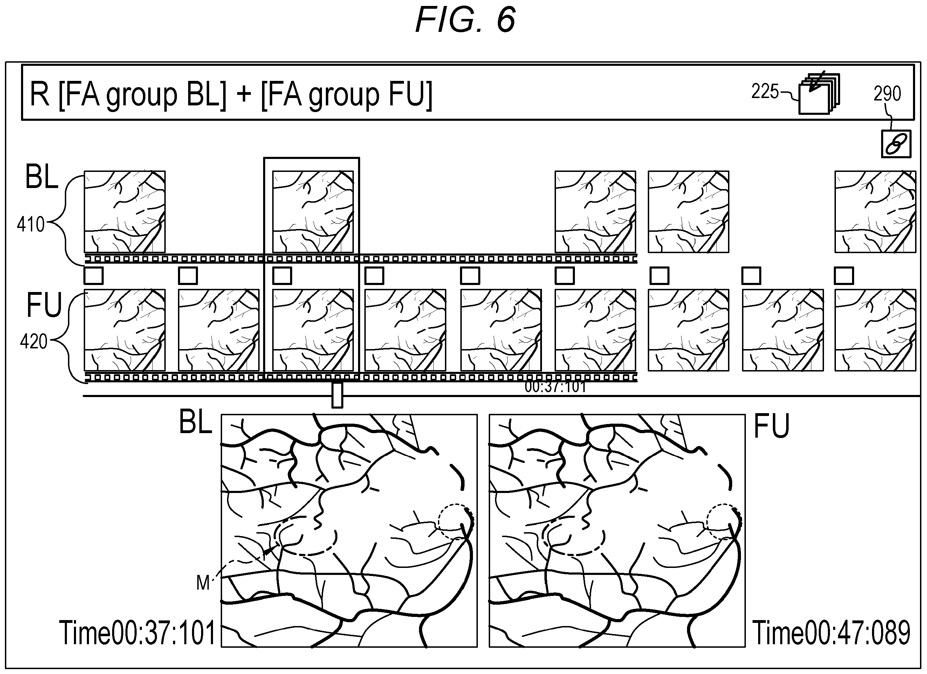

A check screen suitable to select a key frame for a follow-up is illustrated by example as a first modification, using Fig. 6. As a precondition, image group data related to first fluorescent angiography (a baseline) (image group data of the baseline) and key frame specification data is stored in advance in the memory 71. Furthermore, image group data related to second fluorescent angiography (image group data of the follow-up) whose type of angiography is the same as and photographing date is different from (mainly later than) the image group data of the baseline is acquired in advance.

In this case, key frames of the baseline may be displayed on the check screen for the follow-up (the key frame selection screen). The display is based on, for example, the image group data of the baseline and the key frame specification data. Consequently, it is easy to select images suitable for comparisons with the key frames as the key frames of the follow-up.

The key frames of the baseline are arranged and displayed chronologically (as thumbnails in a thumbnail display area 410) on the check screen of Fig. 6. Moreover, fluorescent angiographic images of the follow-up that can be selected as the key frames of the baseline are displayed (as thumbnails) in parallel with the key frames of the baseline (in a thumbnail display area 420).

In this case, the fluorescent angiographic images of the follow-up may be placed, associated with the key frames of the baseline according to the angiography timings. In other words, the placement of each image may be controlled in such a manner as to place thumbnails having the same angiography timing in parallel.

### [Second Modification]

There is no need to set the detailed display area 230 at a predetermined position on the check screen. For example, if the detailed display area 230 is not provided, the thumbnails 211 and 212 are arranged on the check screen, and any of the thumbnails 211 and 212 is selected, a fluorescent angiographic image having a larger size and a higher number of pixels than the selected thumbnail, the fluorescent angiographic image corresponding to the selected thumbnail, may be displayed, switched from the selected thumbnail.

### [Third Modification]

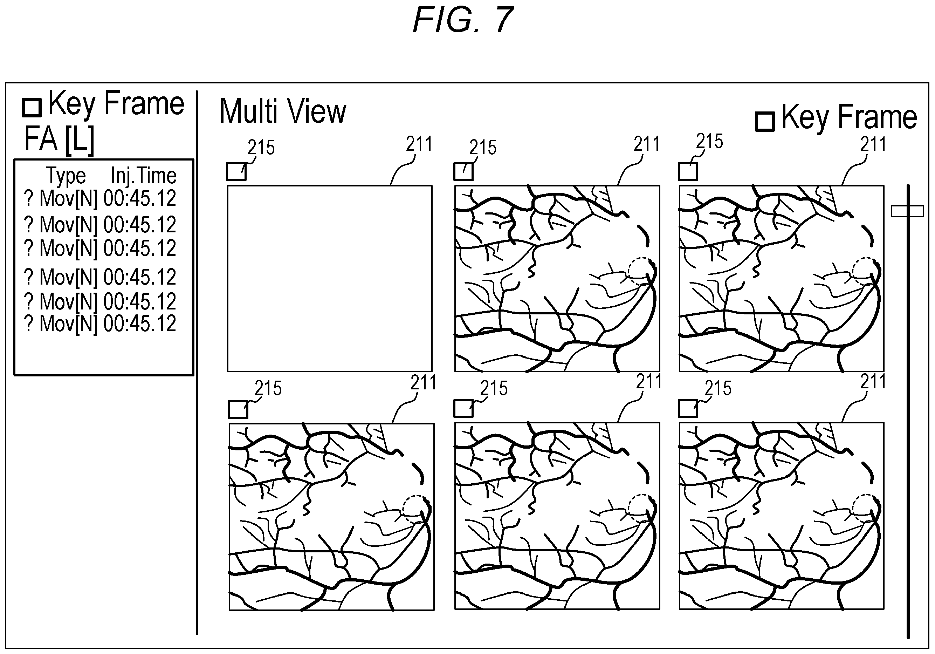

Fig. 7 is one of modifications of the check screen of Fig. 3. Unlike the double display of each fluorescent angiographic image with a thumbnail and an enlarged image in the detailed display area 230 in Fig. 3, a list can be displayed using images of an approximately middle size between the thumbnail and the enlarged image.

### [Fourth Modification]

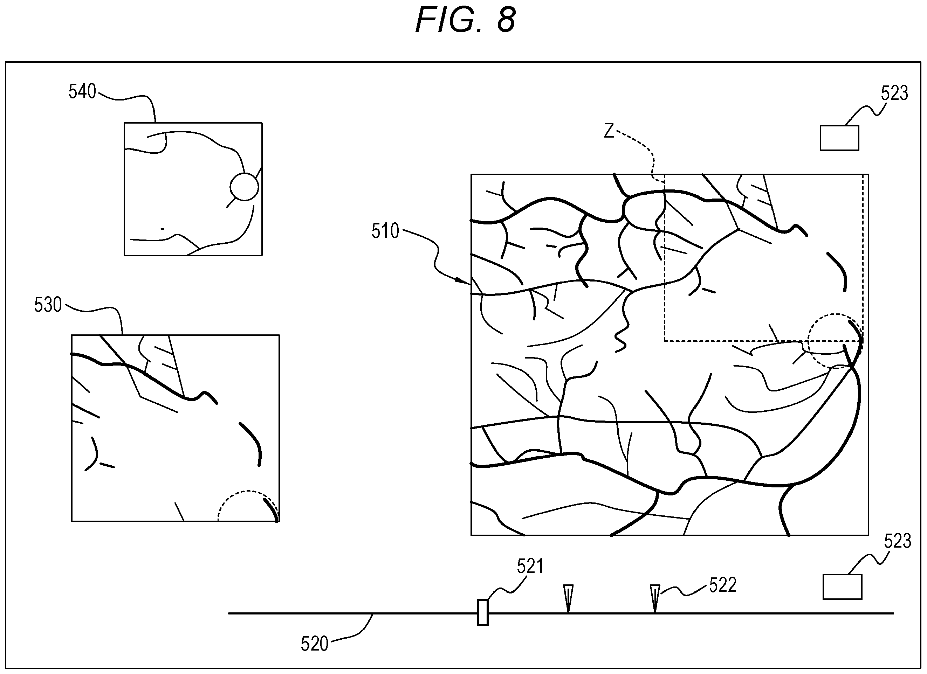

Moreover, it is not necessarily required to display the thumbnails 211 and 212 on the check screen. In the third modification of the check screen illustrated in Fig. 8, at least an image display area 510 and a widget 520 may be included. One fluorescent angiographic image corresponding to the angiography timing selected by the widget 520 is selected from image group data and displayed in the image display area 510.

In the example of Fig. 8, the widget 520 is a slider. An index 522 indicating the angiography timing of an image selected in advance as a key frame is displayed on the slider. A key frame registration button 523 is operated to select a fluorescent angiographic image displayed in the image display area 510 at this point in time as a key frame. At the same time, the index 522 is additionally generated and displayed at the position of a lever 521 of the slider at this point in time.

An enlarged display area 530 is an area for displaying a region of interest on a fluorescent angiographic image displayed in the image display area 510 at a higher magnification than the image display area 510. A region displayed in the enlarged display area 530 is highlighted with a marker Z in the image display area 510. Moreover, a reflection image photographed simultaneously with the fluorescent angiographic image displayed in the image display area 510 is displayed in an observation image display area 540.

### <Summary Edition Screen>

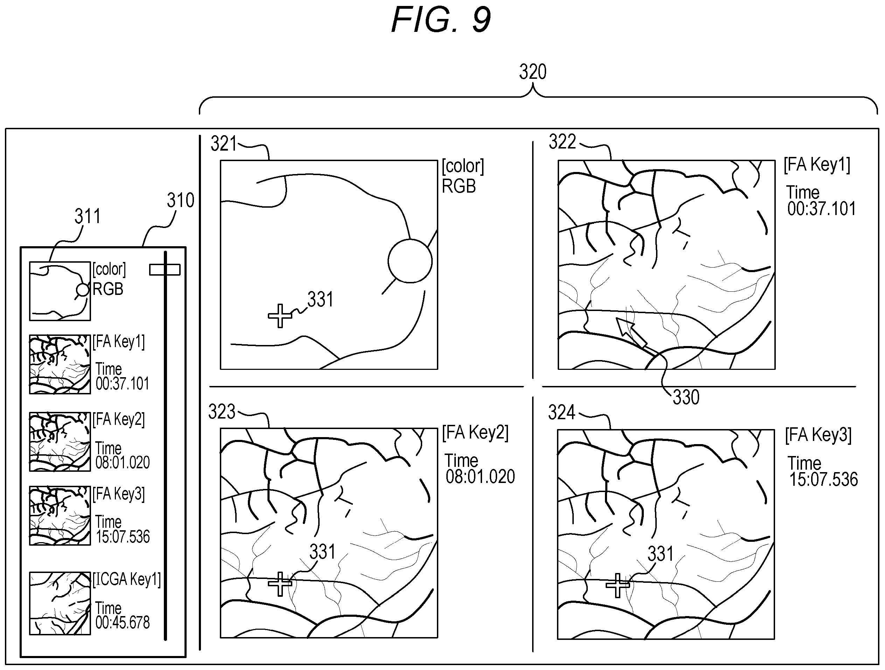

A summary edition screen illustrated in Fig. 9 is used to create a summary related to fluorescent angiography. The summary indicated here includes at least a fundus image and information related to the fundus image. The summary is used to observe and diagnose a fundus disease and the like.

In the embodiment, the summary including at least one or more fluorescent angiographic images is created through the summary edition screen (a summary edition step).

In the embodiment, the summary edition screen is broadly divided into a selection box 310 (a selection area in the embodiment) and a layout area 320 (a summary edition area in the embodiment). Fundus images that can be placed in the layout area 320 are listed in the selection box 310. At least one or more fundus images in the selection box 310 can be selected as an image(s) presented in the summary via the operating unit 75.

The summary is created in the layout area 320. The created summary is saved as a summary file in the memory 71. When the saved summary file is read out, the contents of the layout area 320 (the summary edition area) are displayed as the summary (a summary output step). Moreover, when the created summary is printed, the contents of the layout area 320 are printed. When the summary is displayed or printed, it is simply required to output only the contents of the layout area 320. There is no need to output the selection box 310.

It is desired to place only images illustrating contrast medium appearances useful for observation and diagnosis among a plurality of fluorescent angiographic images obtained through the fluorescent imaging procedure on the summary edition screen to create the summary quickly. Hence, in the embodiment, in terms of fluorescent angiographic images, images selected in advance as key frames are listed in the selection box 310.

In addition, other fundus front images may be listed in the selection box 310. For example, at least one of a reflection image of an examinee's eye and an autofluorescence image may be listed together with the above-described key frames. Both the reflection image and the autofluorescence image may be still images obtained by singly photographing the examinee's eye.

A thumbnail of each fundus front image and information on the fundus front image displayed as the thumbnail are associated and arranged in the selection box 310 in Fig. 9. The information on the fundus front image includes at least information on the photographing method. In a case of a fluorescent angiographic image, identification information indicating the type of angiography (for example, FA and ICGA) is included as the information on the photographing method. Moreover, information indicating the photographing method of a fundus front image obtained by color photography, infrared photography, photography with single color of visible light, autofluorescence photography, or the like may be presented as the information on the photographing method. Moreover, in addition, information indicating the wavelength range of light projected onto the fundus may be presented as the information on the photographing method.

Moreover, the information on the fundus front image may include information indicating a photographing date and time. Moreover, in a case of a thumbnail of a fluorescent angiographic image, time information indicating an angiography timing may be further included.

In the embodiment, the information on the fundus front image displayed as the thumbnail is always displayed near the thumbnail 311. However, it is simply required to associate and display the thumbnail 311 and the information on the fundus front image. For example, it may be a mode in which only when the cursor is placed on the thumbnail 311, the information on the fundus front image presented by the thumbnail 311 is temporarily displayed. It is conceivable that always-on display is easier to select a desired image more quickly than temporary display.

Sub areas whose positions and sizes are predetermined are provided in the layout area 320. In Fig. 9, four sub areas 321 to 324 are provided in one layout area 320. In the example of Fig. 9, most of the layout area 320 is equally divided by the sub areas 321 to 324.

Each of the sub areas 321 to 324 includes an area where a fundus front image is placed (an image placement area), and an area where information on the fundus front image is displayed (a related information display area). As illustrated in Fig. 9, the size of the area where a fundus front image is placed may be equal in the sub areas 321 to 324.

Sub areas for at least three images may be ensured in the layout area 320 (refer to Fig. 9). A reflection image of the examinee's eye, and a fluorescent angiographic image photographed in the early stage of the angiography and a fluorescent angiographic image photographed in the middle or later stage of the angiography in the fluorescent imaging procedure may be able to be placed in the sub areas for three images, respectively. Furthermore, it is preferable to ensure sub areas for four images in such a manner as to be able to display a fluorescent angiographic image photographed in the middle stage of the angiography and a fluorescent angiographic image photographed in the later stage of the angiography in different areas, respectively.

Moreover, the number of sub areas may be changeable on the basis of an operation on the operating unit 75, as appropriate. However, in the embodiment, even if the number of sub areas is increased/reduced, at least the size (the size at the time of storage or printing) of a first area of each sub area is maintained constant. If the image size is constant in each summary, a comparison of summaries can be excellently made. If all sub areas cannot be printed in one sheet due to an increase in number of sub areas, it is preferable to divide the sub areas into a plurality of frames and print them without changing the print scale.

Fundus front images selected via the selection box 310 are displayed in a size larger than a thumbnail in the layout area 320. One of the fundus front images is placed and displayed in the image placement area of each of the sub areas 321 to 324. In addition, information on the fundus front image placed in the image placement area is placed and displayed in the related information display area of each of the sub areas 321 to 324. When a fluorescent angiographic image is placed in the first area, information indicating at least one of the type of angiography and the angiography timing of the fluorescent angiographic image is, for example, placed over or near the fluorescent angiographic image. The placement of such information allows for excellent observation and diagnosis.

The operation of placing fundus front images in the sub areas 321 to 324 may be, for example, a technique that drops the thumbnail 311 in the selection box 310 in each sub area, or another technique.

The above-mentioned information on a fundus front image is placed. Consequently, observation and diagnosis can be excellently performed.

### [Cursor Link]

The fundus front images displayed in the sub areas are aligned and displayed. A fluorescent angiographic image or autofluorescence image is aligned on the basis of a reflection image photographed simultaneously with the fluorescent angiographic image or autofluorescence image. For example, a fundus front image in a sub area may be aligned with reference to another fundus front image in another sub area where the cursor (pointer) of the pointing device is placed. Moreover, an image as a reference for alignment may be determined on the basis of information on a fundus front image such as the photographing date and time and the photographing method. For example, when a reflection image is displayed in any of the sub areas, alignment may be adjusted with reference to the reflection image. In other words, in this case, displacements of the other images with reference to the reflection image are determined, and the alignment is performed.

In the embodiment, the cursor that moves in accordance with the operation of the pointing device may be placed on a fundus front image in a sub area. In this case, a corresponding cursor indicating the same position as the cursor may be placed on a fundus front image in another sub area. Consequently, it becomes easy for the examiner to observe and diagnose a location of interest.

### <Multi-display>

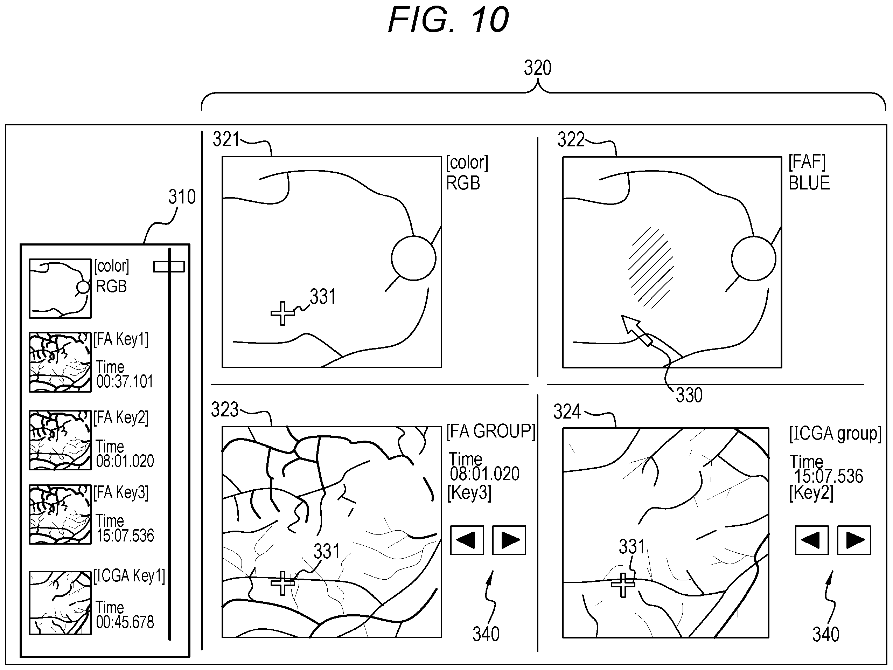

Moreover, as illustrated in Fig. 10, at least three images - a reflection image of the examinee's eye, a fluorescent angiographic image of a first type of angiography (a first fluorescent angiographic image), and a fluorescent angiographic image of a second type of angiography (a second fluorescent angiographic image) - may be simultaneously displayable in the layout area 320. Furthermore, it is preferable to be capable of simultaneously displaying an autofluorescence image. In this case, a switching widget 340 (a switching button in Fig. 10) is placed, associated with each of sub areas where the first and second fluorescent angiographic images are placed. The switching widget 340 is operated to switch the first or second fluorescent angiographic image to another image of the same type of angiography obtained on the same photographing date, or by the same type of fluorescent angiography, and display the other image. In the embodiment, an image displayed is switched by a switching operation in one action to an image having elapsed time closest to the elapsed angiography time of the image displayed among images before and after the elapsed angiography time of the image displayed. An image to be newly displayed is selected here from the other key frames. Consequently, it is possible to display a desired contrast medium appearance according to the type of angiography, and visually compare the contrast medium appearances.

### <Follow-up Display>

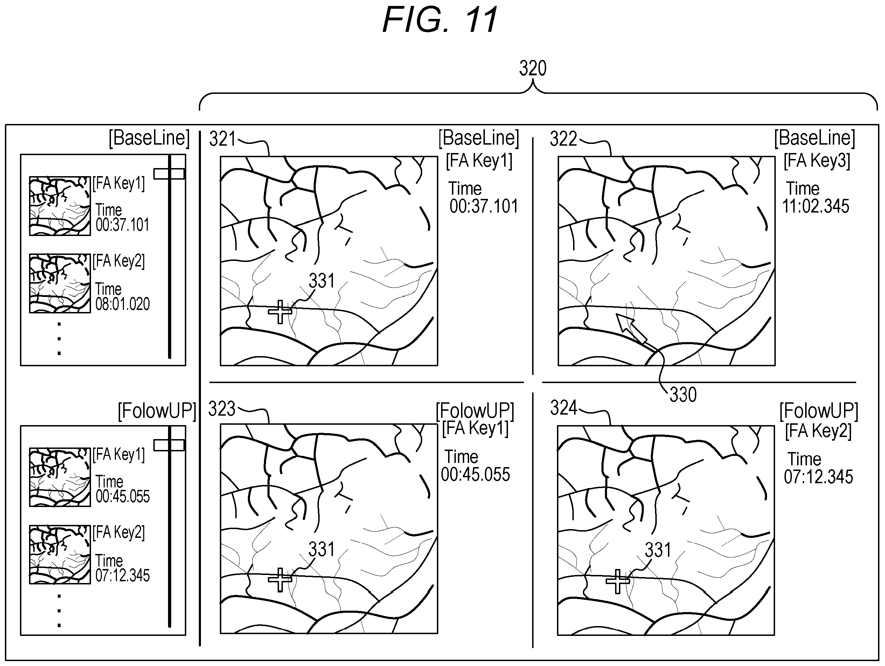

Moreover, as illustrated in Fig. 11, a plurality of fluorescent angiographic images that is the same in type of angiography and different in photographing date may be simultaneously displayable in the layout area 320. In the example of Fig. 11, fluorescent angiographic images by FA as baselines are displayed in the sub areas 321 and 322 in the upper half of the layout area 320, and fluorescent angiographic images by FA photographed at a later date than the baselines are displayed in the sub areas 323 and 324 in the lower half.

In this manner, in Fig. 11, rows including a plurality of sub areas arranged in a first direction are arranged in parallel in a direction crossing the first direction. The placement of fluorescent angiographic images of baselines and fluorescent angiographic images that are the same in type of angiography as and have been photographed on a different photographing date from the baselines (the fluorescent angiographic images of a follow-up) is restricted in such a manner to place the fluorescent angiographic images of the baselines in sub areas in one row and the fluorescent angiographic images of the follow-up in sub areas in the other row. Moreover, as illustrated in Fig. 11, the baseline and the follow-up image may be selected from different selection boxes, respectively.

When a fluorescent angiographic image to serve as a baseline is placed in one of the sub areas 321 and 322 in the upper row, a follow-up image indicating the same angiography timing as the fluorescent angiographic image may be placed automatically in a sub area adjacent to the sub area where the baseline is placed among the sub areas 323 and 324 in the lower row. In other words, a baseline image and a follow-up image, which are the same in angiography timing, are placed in each of pairs of the sub areas 321 and 323, and the sub areas 322 and 324. Moreover, the placement is not limited to the automatic placement, and an image indicating the same angiography timing as the baseline placed in one of the sub areas 321 and 322 may be presented in the selection box 310 as an image that can be placed in a sub area adjacent to the one of the sub areas 321 and 322.

In terms of the displays of Figs. 9 to 11, and the display of the summary screen, each fluorescent angiographic image may be displayed on the basis of at least any of data of a still angiographic image, data of a photographed image group, and key frame specification information, which are stored in advance in the memory 71.

The description has been given above on the basis of the embodiment. However, the present disclosure is not limited to the above-described embodiment, and permits various modifications to the embodiment.

It is conceivable, for example, that single photography in a fluorescent imaging procedure is performed at an angle of view or fixation position different from continuous angiography. Moreover, it is conceivable that single photography is performed as stereo photography (a plurality of images with parallax) unlike continuous angiography. When still angiographic images obtained by such single photography are acquired, the computer 1 may exclude these images from key frame selection targets, for example, display targets on the check screen. Moreover, when an image of an angle of view larger than a frame image obtained by continuous angiography, the image including a photographing area of the frame image, is obtained as a still angiographic image in single photography, the image may be included in the key frame selection targets. For example, it may be configured in such a manner that the photographing area of a frame image is extracted from a still angiographic image of a large angle of view and the extracted image can be displayed on the key frame selection screen.

Moreover, the fundus image observation program of the present disclosure not only is applied to fluorescent angiography, but also can be applied in a case where a desired image is extracted from many photographed images of an examinee's eye acquired as a result of outputting a photographing trigger signal a plurality of times in a photographing session. For example, there are wire-ranging applicable images of the examinee's eye. Specific examples of the applicable images include an OCT image, a functional OCT image, a fundus image, and an anterior segment image. These images may be images at a cell level. The fundus image observation program of the present disclosure may be used, for example, to extract a desired image from images at the cell level photographed chronologically to grasp movements in the images of the examinee's eye (for example, retinal cell images) photographed at the cell level. Moreover, the fundus image observation program of the present disclosure may be used when a drug is administered and chronological reactions to the administration in the examinee's eye are evaluated.

The fundus image observation program of the embodiment may be the following first to thirteenth fundus image observation programs.

The first fundus image observation program is a fundus image observation program that at least displays a fluorescent angiographic image being a fundus front image by fluorescence photographed in fluorescent angiography with a fundus photographing apparatus, the first fundus image observation program causing a processor of a computer to execute: a first acquisition step of acquiring image group data including the fluorescent angiographic images of a plurality of frames acquired through a fluorescent imaging procedure for an examinee's eye; a second acquisition step of selecting and setting at least one or more frames illustrating a desired contrast medium appearance as a key frame(s) from the fluorescent angiographic images included in the image group data and accordingly obtaining key frame specification information being information for specifying the key frames in the image group data; and a list display step of extracting the key frames from the image group data on the basis of the key frame specification information acquired in advance, and displaying the extracted key frames in list form on a display unit.

The second fundus image observation program is the first fundus image observation program wherein in the first acquisition step, the image group data is acquired which includes at least a photographed image group being the fluorescent angiographic images of a plurality of frames photographed continuously at regular intervals, and in the second acquisition step, the key frame specification information is acquired which specifies, as the key frame or a candidate therefor, the fluorescent angiographic image photographed at an output timing of a third trigger signal emitted while the photographed image group is being photographed.

The third fundus image observation program is the second fundus image observation program wherein in the first acquisition step, the image group data is acquired which includes the photographed image group and a still image of the fluorescent angiographic image photographed singly whenever a photographing trigger signal is output, and in the second acquisition step, the key frame specification information of the photographed image group is acquired and also the key frame specification information that specifies all the still images included in the image group data as the key frames or candidates therefor.

The fourth fundus image observation program is any one of the first to third fundus image observation programs wherein in the second acquisition step, a key frame selection screen for selecting the key frames from the fluorescent angiographic images of the plurality of frames included in the image group data on the basis of an examiner's selection operation is displayed on the display unit, at least one or more frames of the fluorescent angiographic images included in the image group data are displayed on the key frame selection screen, and the key frame specification information is acquired which specifies at least one or more fluorescent angiographic images as the key frame(s) in accordance with the selection operation among the fluorescent angiographic images displayed on the key frame selection screen.

The fifth fundus image observation program is the fourth fundus image observation program wherein in the second acquisition step, a selection operation of selecting an angiography timing is accepted via an input interface of the computer, and the fluorescent angiographic image photographed at the angiography timing selected by the selection operation is extracted on the basis of the image group data, and displayed on the key frame selection screen.

The sixth fundus image observation program is the fourth or fifth fundus image observation program wherein in the first acquisition step, the image group data is acquired which includes at least the photographed image group being the fluorescent angiographic images of the plurality of frames photographed continuously at regular intervals, and in the second acquisition step, a widget for selecting a photographing time interval, a frame interval, or an extraction number of frame images extracted from the photographed image group is displayed on the key frame selection screen, and the fluorescent angiographic images extracted at photographing time intervals, at frame intervals, or in the extraction number on the basis of an input operation via the widget are displayed on the key frame selection screen.

The seventh fundus image observation program is any one of the fourth to sixth fundus image observation programs wherein in the first acquisition step, the image group data is acquired which includes at least the photographed image group being the fluorescent angiographic images of the plurality of frames photographed continuously at regular intervals, and in the second acquisition step, the photographed image group is switched and displayed on the key frame selection screen, and also a fourth trigger signal emitted during the switching and display is accepted to obtain the key frame specification information that specifies, as the key frame, the fluorescent angiographic image displayed at an output timing of the fourth trigger signal.

The eighth fundus image observation program is any one of the fourth to seventh fundus image observation programs causing the computer to execute a displacement correction step of correcting displacements of photographing positions of the fluorescent angiographic images included in the image group data, wherein in the second acquisition step, the fluorescent angiographic image after the displacement correction is displayed on the key frame selection screen.

The ninth fundus image observation program is any one of the fourth to eighth fundus image observation programs wherein in the second acquisition step, thumbnails of the fluorescent angiographic images are arranged and displayed in photographing order in a thumbnail display area of the key frame selection screen, and when any of the thumbnails displayed in the thumbnail display area is selected as a detailed display target, the fluorescent angiographic image corresponding to the selected thumbnail is displayed in a larger size or with a higher number of pixels than the thumbnail.

The tenth fundus image observation program is any one of the first to ninth fundus image observation programs wherein in the list display step, when the input of a predetermined operation on a first key frame being any of the key frames displayed on the display unit is accepted via the input interface of the computer, frames included in the image group data other than the key frames, the frames being within a predetermined period from an angiography timing of the first key frame, are displayed on the display unit on the basis of the image group data.

The eleventh fundus image observation program is the tenth fundus image observation program causing the computer to further execute a reselection step of when in the list display step, the frames within the predetermined period from the angiography timing of the first key frame are displayed on the basis of the input of the predetermined operation, selecting and setting any of the frames within the predetermined period as a new key frame on the basis of a second selection operation via the input interface of the computer.

The twelfth fundus image observation program is any one of the first to eleventh fundus image observation programs causing the computer to further execute a determination step of determining whether or not each of the fluorescent angiographic images of the plurality of frames included in the image group data is a failed photographed image photographed at a timing when a blink or fixation disparity occurs, wherein in the second acquisition step, the key frame specification information is acquired from which the failed photographed image among the fluorescent angiographic images of the plurality of frames included in the image group data is excluded from the key frames and candidates therefor.

The thirteenth fundus image observation program is any one of the first to twelfth fundus image observation programs wherein the image group data acquired in the first acquisition step includes elapsed time information indicating time elapsed in the fluorescent imaging procedure, the time elapsing at a photographing timing of each frame included in the image group data, and in the list display step, the elapsed time is displayed, associated with each key frame displayed in list form on the display unit on the basis of the elapsed time information.

The foregoing detailed description has been presented for the purposes of illustration and description. Many modifications and variations are possible in light of the above teaching. It is not intended to be exhaustive or to limit the subject matter described herein to the precise form disclosed. Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims appended hereto.

## Claims

1. A method for fundus image observation, the method using a processor of a computer, the method comprising:
a first acquisition step of acquiring image group data including fluorescent angiographic images of a plurality of frames acquired through a fluorescent imaging procedure for an examinee's eye;
a second acquisition step of selecting and setting at least one or more frames illustrating a desired contrast medium appearance as at least one or more key frames from the fluorescent angiographic images included in the image group data and obtaining key frame specification information for specifying the at least one or more key frames in the
image group data;
a list display step of displaying the key frames extracted from the image group data in list form on a display unit (80) on the basis of the key frame specification information,
wherein, in the first acquisition step, the image group data is acquired which includes at least a photographed image group being the fluorescent angiographic images of a plurality of frames photographed continuously at regular intervals,
the image group data further includes one of the fluorescent angiographic images photographed at an output timing of a third trigger signal emitted during photographing of the photographed image data, and
in the second acquisition step, the fluorescent angiographic image photographed at the output timing of the third trigger signal, whereby the fluorescent
angiographic image being included in the photographed image group, is selected and set as the at least one or more key frames
or a candidate therefor.

2. The method according to claim 1, wherein
the image group data includes the photographed image group and a still image of the fluorescent angiographic image photographed singly at each output timing of a photographing trigger signal, and
in the second acquisition step, the key frame specification information of the photographed image group is acquired and also all the still images included in the image group data are selected and set as the key frames or candidates therefor.

3. The method according to claim 1 or 2, wherein in the second acquisition step,
a key frame selection screen for selecting the key frames from the fluorescent angiographic images of the plurality of frames included in the image group data on the basis of an examiner's selection operation is displayed on the display unit (80),
at least one or more frames of the fluorescent angiographic images included in the image group data are displayed on the key frame selection screen, and
at least one or more fluorescent angiographic images in accordance with the selection operation are selected and set as the key frames from the fluorescent angiographic images displayed on the key frame selection screen.

4. The method according to claim 3, wherein in the second acquisition step,
a selection operation of selecting an angiography timing via an input interface of the computer is accepted, and
the fluorescent angiographic image photographed at the angiography timing selected by the selection operation is extracted on the basis of the image group data, and displayed on the key frame selection screen.

5. The method according to claim 3 or 4, wherein
in the first acquisition step, the image group data is acquired which includes at least the photographed image group being the fluorescent angiographic images of the plurality of frames photographed continuously at regular intervals, and
in the second acquisition step,
frame images are extracted from the photographed image group,
a widget for selecting a photographing time interval, a frame interval, or an extraction number of the frame images is displayed on the key frame selection screen, and
the fluorescent angiographic images extracted at the photographing time intervals, at the frame intervals, or in the extraction number on the basis of an input operation via the widget are displayed on the key frame selection screen.

6. The method according to any one of claims 3 to 5, wherein
in the first acquisition step, the image group data is acquired which includes at least the photographed image group being the fluorescent angiographic images of the plurality of frames photographed continuously at regular intervals, and
in the second acquisition step, the photographed image group is switched and displayed on the key frame selection screen, and a fourth trigger signal emitted during the switching and display is accepted to select and set, as the key frame, the fluorescent angiographic image displayed at an output timing of the fourth trigger signal.

7. The method according to any one of claims 3 to 6, further comprising causing the computer to execute a displacement correction step of correcting displacements of photographing positions of the fluorescent angiographic images included in the image group data, wherein in the second acquisition step, the fluorescent angiographic image after the displacement correction is displayed on the key frame selection screen.

8. The method according to any one of claims 3 to 7, wherein in the second acquisition step,
thumbnails (211, 212) of the fluorescent angiographic images are arranged and displayed in photographing order in a thumbnail display area (210) of the key frame selection screen, and
upon any of the thumbnails (211, 212) displayed in the thumbnail display area (210) being selected as a detailed display target, the fluorescent angiographic image corresponding to the selected thumbnail (211, 212) is displayed in a larger size or with a higher number of pixels than the selected thumbnail (211, 212).

9. The method according to any one of claims 1 to 8, wherein in the list display step, upon input of a predetermined operation on a first key frame being any of the key frames displayed on the display unit (80) having been accepted via the input interface of the computer, frames included in the image group data other than the key frames, the frames being within a predetermined period from an angiography timing of the first key frame, are displayed on the display unit (80) on the basis of the image group data.

10. The method according to claim 9 causing the computer to further execute a reselection step, wherein in the reselection step, upon in the list display step, the frames within the predetermined period from the angiography timing of the first key frame being displayed on the basis of the input of the predetermined operation, any of the frames within the predetermined period is selected and set as a new key frame on the basis of a second selection operation via the input interface of the computer.

11. The method according to any one of claims 1 to 10, causing the computer to further execute a determination step of determining whether or not each of the fluorescent angiographic images of the plurality of frames included in the image group data is a failed photographed image photographed at a timing of occurrence of a blink or fixation disparity, wherein in the second acquisition step, the failed photographed image among the fluorescent angiographic images of the plurality of frames included in the image group data is excluded from the key frames and candidates therefor.

12. The method according to any one of claims 1 to 11, wherein
the image group data acquired in the first acquisition step includes elapsed time information indicating time elapsed in the fluorescent imaging procedure, the time elapsing at a photographing timing of each frame included in the image group data, and
in the list display step, the elapsed time is displayed, associated with each key frame displayed in list form on the display unit on the basis of the elapsed time information.

13. A memory (71) storing instructions which, when executed by a computer (1), cause the computer (1) to control a method according to any of the preceding claims.

## Patentansprüche

1. Ein Verfahren zur Fundusbildbeobachtung, wobei das Verfahren einen Prozessor eines Computers verwendet, wobei das Verfahren folgende Schritte aufweist:
einen ersten Erfassungsschritt zum Erfassen von Bildgruppendaten, die fluoreszente angiographische Bilder einer Mehrzahl von Rahmen umfassen, die durch einen Fluoreszenzbilderfassungsprozess für ein Auge einer Probanden erfasst werden;
einen zweiten Erfassungsschritt zum Auswählen und Einstellen zumindest eines oder mehrerer Rahmen, die ein gewünschtes Kontrastmittelerscheinungsbild veranschaulichen, als zumindest einen oder mehrere Schlüsselrahmen aus den fluoreszenten angiographischen Bildern, die in den Bildgruppendaten enthalten sind, und Erhalten von Schlüsselrahmenspezifikationsinformationen zum Spezifizieren des zumindest einen oder der mehreren Schlüsselrahmen in den Bildgruppendaten;
einen Listenanzeigeschritt zum Anzeigen der aus den Bildgruppendaten extrahierten Schlüsselrahmen in Listenform auf einer Anzeigeneinheit (80) auf der Basis der Schlüsselrahmenspezifikationsinformationen,
wobei in dem ersten Erfassungsschritt die Bildgruppendaten erfasst werden, die zumindest eine Fotografiertes-Bild-Gruppe umfassen, welche die fluoreszenten angiographischen Bilder einer Mehrzahl von Rahmen sind, die fortlaufend bei regelmäßigen Abständen fotografiert werden,
wobei die Bildgruppendaten ferner eines der fluoreszenten angiographischen Bilder umfassen, die zu einem Ausgangszeitpunkt eines während des Fotografierens der fotografierten Bilddaten ausgesendeten dritten Auslösesignals fotografiert werden, und
in dem zweiten Erfassungsschritt das fluoreszente angiographische Bild, das zu dem Ausgangszeitpunkt des dritten Auslösesignals fotografiert wird, wobei das fluoreszente angiographische Bild, das in der Fotografiertes-Bild-Gruppe enthalten ist, ausgewählt und als der zumindest eine oder die mehreren Schlüsselrahmen oder als ein Kandidat davon eingestellt wird.

2. Das Verfahren gemäß Anspruch 1, wobei
die Bildgruppendaten die Fotografiertes-Bild-Gruppe und ein Standbild des fluoreszenten angiographischen Bildes umfassen, welches einmalig bei jedem Ausgangszeitpunkt eines Fotografie-Auslösesignals fotografiert wird, und
in dem zweiten Erfassungsschritt die Schlüsselrahmenspezifikationsinformationen der Fotografiertes-Bild-Gruppe erfasst werden und außerdem alle Standbilder, die in den Bildgruppendaten enthalten sind, ausgewählt werden und als die Schlüsselrahmen oder Kandidaten davon festgelegt werden.

3. Das Verfahren gemäß Anspruch 1 oder 2, wobei in einem zweiten Erfassungsschritt
ein Schlüsselrahmenauswahlbildschirm zum Auswählen der Schlüsselrahmen aus den fluoreszenten angiographischen Bildern der Mehrzahl von Rahmen, die in den Bildgruppendaten enthalten sind, auf der Basis eines Auswahlvorganges einer Prüfperson auf der Anzeigeeinheit (80) angezeigt wird,
zumindest ein oder mehrere Rahmen der fluoreszenten angiographischen Bilder, die in den Bildgruppendaten enthalten sind, auf dem Schlüsselrahmenauswahlbildschirm angezeigt werden, und
zumindest ein oder mehrere fluoreszente angiographische Bilder gemäß dem Auswahlvorgang aus den fluoreszenten angiographischen Bildern, die auf dem Schlüsselrahmenauswahlbildschirm angezeigt werden, ausgewählt werden und als die Schlüsselrahmen eingestellt werden.

4. Das Verfahren gemäß Anspruch 3, wobei in dem zweiten Erfassungsschritt,
ein Auswahlvorgang zum Auswählen eines Angiographiezeitpunktes über eine Eingabeschnittstelle des Computers akzeptiert wird, und
das fluoreszente angiographische Bild, das bei dem durch den Auswahlvorgang ausgewählten Angiographiezeitpunkt fotografiert wird, auf der Basis der Bildgruppendaten extrahiert wird, und auf dem Schlüsselrahmenauswahlbildschirm angezeigt wird.

5. Das Verfahren gemäß Anspruch 3 oder 4, wobei
in dem ersten Erfassungsschritt die Bildgruppendaten erfasst werden, die zumindest die Fotografiertes-Bild-Gruppe umfassen, welche die fluoreszenten angiographischen Bilder der Mehrzahl von Rahmen sind, die fortlaufend bei regelmäßigen Abständen fotografiert werden, und
in dem zweiten Erfassungsschritt,
Rahmenbilder aus der Fotografiertes-Bild-Gruppe extrahiert werden,
ein Grafikobjekt zum Auswählen eines Fotografiezeitintervalls, eines Rahmenintervalls oder einer Extraktionsanzahl der Rahmenbilder auf dem Schlüsselrahmenauswahlbildschirm angezeigt wird, und
die fluoreszenten angiographischen Bilder, die zu den Fotografiezeitintervallen, den Rahmenintervallen oder mit der Extraktionsanzahl auf der Basis einer Eingabeoperation über das Grafikobjekt extrahiert werden, auf dem Schlüsselrahmenauswahlbildschirm angezeigt werden.

6. Das Verfahren gemäß einem der Ansprüche 3 bis 5, wobei
in dem ersten Erfassungsschritt die Bildgruppendaten erfasst werden, die zumindest die Fotografiertes-Bild-Gruppe umfassen, welche die fluoreszenten angiographischen Bilder der Mehrzahl von Rahmen sind, die fortlaufend bei regelmäßigen Abständen fotografiert werden, und
in dem Erfassungsschritt die Fotografiertes-Bild-Gruppe getauscht und auf dem Schlüsselrahmenauswahlbildschirm angezeigt wird, und ein während des Tauschens und Anzeigens ausgesendetes viertes Auslösesignal dahin gehend akzeptiert wird, das bei einem Ausgangszeitpunk des vierten Auslösesignals angezeigte fluoreszente angiographische Bild auszuwählen und als den Schlüsselrahmen festzulegen.

7. Das Verfahren gemäß einem der Ansprüche 3 bis 6, das ferner ein Bewirken dahingehend aufweist, dass der Computer einen Verschiebungskorrekturschritt zum Korrigieren von Verschiebungen von Fotografiepositionen der in Bildgruppendaten enthaltenen fluoreszenten angiographischen Bilder ausführt, wobei in dem zweiten Erfassungsschritt die fluoreszenten angiographischen Bilder nach der Verschiebungskorrektur auf dem Schlüsselrahmenauswahlbildschirm angezeigt werden.

8. Das Verfahren gemäß einem der Ansprüche 3 bis 6, wobei in dem zweiten Erfassungsschritt
Miniaturbilder (211, 212) der fluoreszenten angiographischen Bilder in einer Fotografiereihenfolge in einem Miniaturbildanzeigebereich (210) des Schlüsselrahmenauswahlbildschirms angeordnet oder angezeigt werden, und
daraufhin, dass eines der in dem Miniaturbildanzeigebereich (210) angezeigten Miniaturbilder (211, 212) als ein Detailanzeigeziel ausgewählt wird, das dem ausgewählten Miniaturbild (211, 212) entsprechende fluoreszente angiographische Bild in größerer Größe oder mit einer höheren Anzahl von Pixeln angezeigt wird als das ausgewählte Miniaturbild (211, 212).

9. Das Verfahren gemäß einem der Ansprüche 1 bis 8, wobei in dem Listenanzeigeschritt daraufhin, dass eine Eingabe eines vorbestimmten Vorganges an einem ersten Schlüsselrahmen, welcher einer der auf der Anzeigeeinheit (80) angezeigten Schlüsselrahmen ist, über die Eingangsschnittstelle des Computers akzeptiert worden ist, Rahmen, die außer den Schlüsselrahmen in den Bildgruppendaten enthalten sind, wobei die Rahmen in einem vorbestimmten Zeitraum von einem Angiographiezeitpunkt des ersten Schlüsselrahmens liegen, auf der Anzeigeeinheit (80) auf der Basis der Bildgruppendaten angezeigt werden.

10. Das Verfahren gemäß Anspruch 9, das bewirkt, dass der Computer ferner einen Wiederauswahlschritt ausführt, wobei in dem Wiederauswahlschritt daraufhin, dass in dem Listenanzeigeschritt die Rahmen in dem vorbestimmten Zeitraum von dem Angiographiezeitpunkt des ersten Schlüsselrahmens auf der Basis der Eingabe der vorbestimmten Operation angezeigt werden, einer der Rahmen in dem vorbestimmten Zeitraum ausgewählt und als neuer Schlüsselrahmen auf der Basis eines zweiten Auswahlvorganges über die Eingangsschnittstelle des Computers festgelegt wird.

11. Das Verfahren gemäß einem der Ansprüche 1 bis 10, das bewirkt, dass der Computer ferner einen Bestimmungsschritt zum Bestimmen ausführt, ob jedes der fluoreszenten angiographischen Bilder der Mehrzahl von Rahmen, die in den Bildgruppendaten enthalten sind, ein fehlgeschlagenes fotografiertes Bild ist, das zu einem Zeitpunkt eines Auftretens eines Lidschlages oder einer Fixierungsdisparität fotografiert wird, oder nicht, wobei in dem zweiten Erfassungsschritt das fehlgeschlagene fotografierte Bild aus den fiuoreszenten angiographischen Bildern der Mehrzahl von Rahmen, die in den Bildgruppendaten enthalten sind, aus den Schlüsselrahmen und Kandidaten davon ausgeschlossen wird.

12. Das Verfahren gemäß einem der Ansprüche 1 bis 11, wobei
die in dem ersten Erfassungsschritt erfassten Bildgruppendaten Informationen über verstrichene Zeit umfassen, welche eine verstrichene Zeit in dem Fluoreszenzbilderfassungsprozess anzeigen, wobei die Zeit bei einem Fotografiezeitpunkt jedes in den Bildgruppendaten enthaltenen Rahmens verstreicht, und
in dem Listenanzeigeschritt die verstrichene Zeit angezeigt wird, wobei dieselbe jedem in der Listenform auf der Anzeigeeinheit angezeigten Schlüsselrahmen auf der Basis der Informationen über verstrichene Zeit zugeordnet ist.

13. Ein Speicher (71), der Anweisungen speichert, die dann, wenn dieselben durch einen Computer (1) ausgeführt werden, bewirken, dass der Computer (1) ein Verfahren gemäß einem der vorhergehenden Ansprüche steuert.

## Revendications

1. Procédé d'observation d'une image de fond d'œil, le procédé utilisant un processeur d'un ordinateur, le procédé comprenant:
une première étape d'acquisition consistant à acquérir les données de groupe d'images comportant les images angiographiques fluorescentes d'une pluralité de trames acquises par une procédure d'imagerie par fluorescence pour l'œil d'une personne examinée;
une deuxième étape d'acquisition consistant à sélectionner et à définir au moins une ou plusieurs trames illustrant un aspect de milieu de contraste souhaité ainsi qu'au moins une ou plusieurs trames clés parmi les images angiographiques fluorescentes incluses dans les données de groupe d'images et à obtenir les informations de spécification de trame clé pour spécifier les au moins une ou plusieurs trames clés dans les données de groupe d'images;
une étape d'affichage de liste consistant à afficher les trames clés extraites des données de groupe d'images sous forme de liste à une unité d'affichage (80) sur base des informations de spécification de trames clés,
dans lequel sont acquises, à la première étape d'acquisition, les données de groupe d'images qui comportent au moins un groupe d'images photographiées qui sont les images angiographiques fluorescentes d'une pluralité de trames photographiées en continu à des intervalles réguliers,
les données de groupe d'images comportent par ailleurs l'une des images angiographiques fluorescentes photographiées à un moment de sortie d'un troisième signal de déclenchement émis pendant la photographie des données d'images photographiées, et
à la deuxième étape d'acquisition, l'image angiographique fluorescente photographiée au moment de la sortie du troisième signal de déclenchement, où l'image angiographique fluorescente est incluse dans le groupe d'images photographiées, est sélectionnée et définie comme au moins une ou plusieurs trames clés ou une candidate à ces dernières.

2. Procédé selon la revendication 1, dans lequel
les données de groupe d'images comportent le groupe d'images photographiées et une image fixe de l'image angiographique fluorescente photographiée individuellement à chaque moment de sortie d'un signal de déclenchement de photographie, et
à la deuxième étape d'acquisition, les informations de spécification de trames clés du groupe d'images photographiées sont acquises et, de même, toutes les images fixes incluses dans les données de groupe d'images sont sélectionnées et définies comme trames clés ou candidates à ces dernières.

3. Procédé selon la revendication 1 ou 2, dans lequel, à la deuxième étape d'acquisition,
un écran de sélection de trames clés pour sélectionner les trames clés des images angiographiques fluorescentes de la pluralité de trames incluses dans les données de groupe d'images sur base d'une opération de sélection de l'examinateur est affiché à l'unité d'affichage (80),
au moins une ou plusieurs trames des images angiographiques fluorescentes incluses dans les données de groupe d'images sont affichées à l'écran de sélection de trames clés, et
au moins une ou plusieurs images angiographiques fluorescentes selon l'opération de sélection sont sélectionnées et définies comme trames clés parmi les images angiographiques fluorescentes affichées à l'écran de sélection de trames clés.

4. Procédé selon la revendication 3, dans lequel, à la deuxième étape d'acquisition,
une opération de sélection consistant à sélectionner un moment d'angiographie par l'intermédiaire d'une interface d'entrée de l'ordinateur est acceptée, et
l'image angiographique fluorescente photographiée au moment d'angiographie sélectionné par l'opération de sélection est extraite sur base des données de groupe d'images, et affichée à l'écran de sélection de trames clés.

5. Procédé selon la revendication 3 ou 4, dans lequel
à la première étape d'acquisition sont acquises les données de groupe d'images qui comportent au moins le groupe d'images photographiées qui sont les images angiographiques fluorescentes de la pluralité de trames photographiées en continu à des intervalles réguliers, et
à la deuxième étape d'acquisition,
les images de trame sont extraites du groupe d'images photographiées,
un widget pour sélectionner un intervalle de temps de photographie, un intervalle de trame ou un nombre d'extractions des images de trame est affiché à l'écran de sélection de trames clés, et
les images angiographiques fluorescentes extraites aux intervalles de temps de photographie, aux intervalles de trames, ou au cours du nombre d'extractions sur base d'une opération d'entrée par l'intermédiaire du widget sont affichées à l'écran de sélection de trames clés.

6. Procédé selon l'une quelconque des revendications 3 à 5, dans lequel
à la première étape d'acquisition sont acquises les données de groupe d'images qui comportent au moins le groupe d'images photographiées qui sont les images angiographiques fluorescentes de la pluralité de trames photographiées en continu à des intervalles réguliers, et
à la deuxième étape d'acquisition, le groupe d'images photographiées est commuté et affiché à l'écran de sélection de trames clés, et un quatrième signal de déclenchement émis pendant la commutation et l'affichage est accepté pour sélectionner et définir, comme trame clé, l'image angiographique fluorescente affichée à un moment de sortie du quatrième signal de déclenchement.

7. Procédé selon l'une quelconque des revendications 3 à 6, comprenant par ailleurs le fait d'amener l'ordinateur à exécuter une étape de correction de déplacement consistant à corriger les déplacements des positions de photographie des images angiographiques fluorescentes incluses dans les données de groupe d'images, dans lequel, à la deuxième étape d'acquisition, l'image angiographique fluorescente après la correction de déplacement est affichée à l'écran de sélection de trames clés.

8. Procédé selon l'une quelconque des revendications 3 à 7, dans lequel, à la deuxième étape d'acquisition,
des images miniatures (211, 212) des images angiographiques fluorescentes sont disposées et affichées dans l'ordre de photographie dans une zone d'affichage d'images miniatures (210) de l'écran de sélection de trames clés, et
lorsque l'une quelconque des images miniatures (211, 212) affichées dans la zone d'affichage d'images miniatures (210) est sélectionnée comme cible d'affichage détaillée, l'image angiographique fluorescente correspondant à l'image miniature sélectionnée (211, 212) est affichée à une grandeur plus grande ou avec un nombre plus élevé de pixels que l'image miniature sélectionnée (211, 212).

9. Procédé selon l'une quelconque des revendications 1 à 8, dans lequel, à l'étape d'affichage de liste, après que l'entrée d'une opération prédéterminée sur une première trame clé qui est l'une quelconque des trames clés affichées à l'unité d'affichage (80) a été acceptée par l'interface d'entrée de l'ordinateur, les trames incluses dans les données de groupe d'images autres que les trames clés, les trames se situant dans les limites d'une période prédéterminée à partir d'un moment d'angiographie de la première trame clé, sont affichées à l'unité d'affichage (80) sur base des données de groupe d'images.

10. Procédé selon la revendication 9, amenant l'ordinateur à exécuter par ailleurs une étape de resélection, dans lequel, à l'étape de resélection, après que, à l'étape d'affichage de liste, les trames se situant dans les limites de la période prédéterminée à partir du moment d'angiographie de la première trame clé qui est affichée sur base de l'entrée de l'opération prédéterminée, l'une quelconque des trames se situant dans les limites de la période prédéterminée est sélectionnée et définie comme nouvelle trame clé sur base d'une deuxième opération de sélection par l'intermédiaire de l'interface d'entrée de l'ordinateur.

11. Procédé selon l'une quelconque des revendications 1 à 10, amenant l'ordinateur à exécuter par ailleurs une étape de détermination consistant à déterminer si chacune des images angiographiques fluorescentes de la pluralité de trames incluses dans les données de groupe d'images est ou non une image photographiée échouée photographiée à un moment de l'occurrence d'un clignement ou d'une disparité de fixation, dans lequel, à la deuxième étape d'acquisition, l'image photographiée échouée parmi les images angiographiques fluorescentes de la pluralité de trames incluses dans les données de groupe d'images est exclue des trames clés et des candidates à ces dernières.

12. Procédé selon l'une quelconque des revendications 1 à 11, dans lequel
les données de groupe d'images acquises à la première étape d'acquisition comportent les informations de temps écoulé indiquant le temps écoulé dans la procédure d'imagerie par fluorescence, le temps qui s'écoule à un moment de photographie de chaque trame incluse dans les données de groupe d'images, et
à l'étape d'affichage de liste est affiché le temps écoulé associé à chaque trame clé affichée sous forme de liste à l'unité d'affichage sur base des informations de temps écoulé.

13. Mémoire (71) mémorisant des instructions qui, lorsqu'elles sont exécutées par un ordinateur (1), amènent l'ordinateur (1) à commander un procédé selon l'une quelconque des revendications précédentes.
